(19) Europäisches Patentamt / European Patent Office / Office européen des brevets

(11) **EP 2 821 786 B1**

(12) **FASCICULE DE BREVET EUROPEEN**

(45) Date de publication et mention de la délivrance du brevet:
**03.09.2025 Bulletin 2025/36**

(51) Classification Internationale des Brevets (IPC):
**G01N 15/1434** (2024.01)  **G01N 33/49** (2006.01)

(21) Numéro de dépôt: **14177673.2**

(52) Classification Coopérative des Brevets (CPC):
**G01N 33/4905; G01N 15/1434; G01N 15/1436;**
G01N 2015/012; G01N 2015/1027;
G01N 2015/1454; G03H 2001/0447

(22) Date de dépôt: **31.05.2013**

(54) **Procédé et système de caractérisation de l'agglutination de particules contenues dans un liquide**

Verfahren und System zur Charakterisierung der Agglutination von in einer Flüssigkeit enthaltenen Teilchen

Method and system for characterisation of the agglutination of particles contained in a liquid

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2012 FR 1255115**

(43) Date de publication de la demande:
**07.01.2015 Bulletin 2015/02**

(62) Numéro(s) de document de la (des) demande(s) initiale(s) en application de l'article 76 CBE:
**13169989.4 / 2 669 678**

(73) Titulaire: **Commissariat à l'Energie Atomique et aux Energies Alternatives**
**75015 Paris (FR)**

(72) Inventeurs:
• **Poher, Vincent**
  **62340 Guines (FR)**
• **Cubizolles, Myriam-Laure**
  **38700 Corenc (FR)**
• **Pouteau, Patrick**
  **38240 Meylan (FR)**
• **Allier, Cédric**
  **38000 Grenoble (FR)**
• **Spiaczka, Johanna**
  **38650 Roissard (FR)**

(74) Mandataire: **INNOV-GROUP**
  **209 Avenue Berthelot**
  **69007 Lyon (FR)**

(56) Documents cités:
**EP-A1- 2 233 923     US-A1- 2006 110 283**

• **DAN CHICEA: "Results of sediment motion visualization by a modified LASCA technique", PROCEEDINGS OF SPIE, S P I E - INTERNATIONAL SOCIETY FOR OPTICAL ENGINEERING, US, vol. 6785, 1 January 2007 (2007-01-01), pages 67851O - 1, XP007918645, ISSN: 0277-786X, DOI: 10.1117/12.757883**
• **SEEMANTINI K NADKARNI ET AL: "Characterization of Atherosclerotic Plaques by Laser Speckle Imaging", CIRCULATION, LIPPINCOTT WILLIAMS & WILKINS, US, vol. 112, 1 January 2005 (2005-01-01), pages 885 - 892, XP007918646, ISSN: 0009-7322, [retrieved on 20050801], DOI: 10.1161/ CIRCULATIONAHA.104.520098**
• **PIEDERRIÈRE Y ET AL: "Evaluation of blood plasma coagulation dynamics by speckle analysis", JOURNAL OF BIOMEDICAL OPTICS, S P I E - INTERNATIONAL SOCIETY FOR OPTICAL ENGINEERING, US, vol. 9, no. 2, 1 March 2004 (2004-03-01), pages 408 - 412, XP002551759, ISSN: 1083-3668, [retrieved on 20040322], DOI: 10.1117/1.1645799**
• **VYACHESLAV KALCHENKO ET AL: "<title>An optical approach for non-invasive blood clot testing</title>", PROCEEDINGS OF SPIE, vol. 6445, 8 February 2007 (2007-02-08), pages 644508 - 644508-8, XP055140996, ISSN: 0277-786X, DOI: 10.1117/12.699491**

- MARKANDEY M. TRIPATHI ET AL: "Assessing blood coagulation status with laser speckle rheology", BIOMEDICAL OPTICS EXPRESS, vol. 5, no. 3, 1 March 2014 (2014-03-01), pages 817 - 831, XP055154538, ISSN: 2156-7085, DOI: 10.1364/BOE.5.000817
- PIEDERRIERE Y ET AL: "Particle aggregation monitoring by speckle size measurement; application to blood platelets aggregation", OPTICS EXPRESS, OSA (OPTICAL SOCIETY OF AMERICA), WASHINGTON DC, (US), vol. 12, no. 19, 20 September 2004 (2004-09-20), pages 4596 - 4601, XP002616137, ISSN: 1094-4087, DOI: 10.1364/OPEX.12.004596
- KALCHENKO V ET AL: "In vivo dynamic light scattering imaging of blood coagulation", JOURNAL OF BIOMEDICAL OPTICS, S P I E - INTERNATIONAL SOCIETY FOR OPTICAL ENGINEERING, US, vol. 12, no. 5, 12 September 2007 (2007-09-12), pages 52002 - 1, XP002551829, ISSN: 1083-3668, [retrieved on 20070912], DOI: 10.1117/1.2778695

**Description**

La présente invention concerne un procédé de caractérisation d'une variation de la vitesse de particules ou d'une agglomération de particules selon la revendication 1 et un système de caractérisation de l'agglutination de particules contenues dans un liquide selon la revendication 12.

L'invention concerne également un système de caractérisation de la variation de la vitesse des particules ou de l'agglomération des particules contenues dans le liquide, telles que des particules sanguines.

L'invention concerne notamment le domaine de l'imagerie sans lentille de focalisation du faisceau lumineux éclairant la chambre fluidique, afin de caractériser un liquide, tel que le sang.

L'invention s'applique en particulier à la détermination d'un paramètre concernant la coagulation du sang, notamment la mesure du temps de coagulation. Elle s'applique également à la détermination d'un paramètre concernant l'agglutination de particules dans le sang, notamment la détermination du groupe sanguin en caractérisant une agrégation cellulaire entre le sang à tester et un anticorps.

On connaît du document EP 2 233 923 A1 un procédé et un système de caractérisation du type précité. Le procédé décrit vise à caractériser la dynamique de coagulation ou de sédimentation d'un fluide contenant du sang. Le système pour la mise en œuvre de ce procédé comprend une chambre fluidique de réception du liquide, une source de lumière spatialement cohérente propre à émettre un faisceau lumineux d'éclairement et un miroir de réflexion du faisceau lumineux en direction de la chambre. Le faisceau lumineux s'étend selon une direction longitudinale depuis le miroir de réflexion vers la chambre fluidique.

Les documents Piederrière Y. et al « Evaluation of blood plasma coagulation dynamics by speckle analysis » et Tripathi M. et al « Assessing blood coagulation status with laser speckle rheology" décrivent des procédés optiques de caractérisation de la coagulation de sang.

Le document Piederrière Y. et al « Particle aggregation monitoring by speckle size measurement ; application to blood platelets aggregation » décrit un procédé permettant de suivre l'aggrégation de plaquettes.

Le système comprend également un capteur d'images, tel qu'un capteur matriciel de type CCD (de l'anglais *Charged-Coupled Device*) ou CMOS (de l'anglais *Complementary Metal Oxyde Semi-conductor*), agencé pour permettre l'acquisition d'une série temporelle d'images d'un motif optique de granularité engendré par l'interaction entre les particules contenues dans la chambre et le faisceau lumineux. Le système de caractérisation comprend également une unité de traitement de ladite série temporelle d'images.

La chambre fluidique est disposée entre le miroir et le capteur d'images selon la direction longitudinale. La distance entre la chambre fluidique et le capteur d'images selon la direction longitudinale est de quelques centimètres ou dizaines de centimètres. Le faisceau lumineux émis par la source de lumière spatialement cohérente présente une surface comprise entre 10 $\mu m^2$ et quelques $mm^2$ suivant un plan perpendiculaire à la direction longitudinale et traversant la chambre fluidique.

Un tel système et un tel procédé permettent de caractériser efficacement la dynamique de coagulation ou de sédimentation du sang contenu dans le liquide.

Toutefois, un tel système est assez encombrant. De plus, il permet d'observer le phénomène de coagulation seulement dans un volume relativement réduit de la chambre fluidique.

Le but de l'invention est donc de proposer un procédé et un système de caractérisation permettant d'observer un plus grand volume de liquide tout en limitant l'encombrement du système de caractérisation.

A cet effet, l'invention a pour objet un procédé de caractérisation selon la revendication 1 annexée

Suivant d'autres aspects avantageux de l'invention, le procédé de caractérisation comprend une ou plusieurs des caractéristiques suivantes, prises isolément ou suivant toutes les combinaisons techniquement possibles :

- le faisceau lumineux présente une surface comprise entre 5 $mm^2$ et 200 $mm^2$, de préférence égale à 25 $mm^2$, suivant un plan perpendiculaire à la direction longitudinale, ledit plan étant agencé au contact de la chambre fluidique ;

  - le faisceau lumineux éclaire directement la chambre fluidique, et l'image est formée directement par le rayonnement transmis par la chambre fluidique éclairée, en l'absence d'une optique de grossissement disposée entre la chambre fluidique et le photodétecteur ; Cela n'exclut pas l'utilisation possible de microlentilles de focalisation au disposées au niveau de chaque pixel du photodétecteur ;

  - la source de lumière est une source de lumière spatialement et temporellement cohérente, telle qu'un laser ;

- le procédé comporte en outre une étape de mélange du liquide avec un réactif apte à engendrer une agglomération des particules, dans lequel, lors de l'étape de calcul, un deuxième indicateur calculé est un indicateur pour chaque image acquise, le deuxième indicateur étant représentatif de l'intensité des pixels de l'image dans une région prédéterminée de l'image et dans lequel le procédé comporte en outre une étape de détermination d'un état d'agglomération des particules à partir du deuxième indicateur calculé ;

- l'état d'agglomération est déterminé lorsque le deuxième indicateur dépasse un seuil prédéterminé ;
- l'état d'agglomération est déterminé lorsque le deuxième indicateur dépasse un indicateur de référence, obtenu par une image réalisée dans une zone de référence ;
- les particules sanguines sont des globules rouges, le réactif comporte un anticorps, et une information relative au groupe sanguin est en outre déterminée à partir de l'état d'agglomération ;
- le liquide comporte un analyte, le procédé comportant alors l'estimation de la quantité dudit analyte dans le liquide, en fonction dudit deuxième indicateur ; et
- la chambre fluidique comporte plusieurs canaux de circulation du fluide, et dans lequel, lors de l'étape de calcul, un indicateur est calculé pour chacun des canaux.

L'invention a également pour objet un système selon la revendication 13 annexée.

Suivant un autre aspect avantageux de l'invention, le photodétecteur matriciel comporte une pluralité de pixels, chaque pixel présentant des dimensions chacune inférieures ou égales à 4 $\mu$m.

Ces caractéristiques et avantages de l'invention apparaîtront à la lecture de la description qui va suivre, donnée uniquement à titre d'exemple non limitatif, et faite en référence aux dessins annexés, sur lesquels :

- la figure 1 est une représentation très schématique d'un système de caractérisation selon l'invention, comprenant une chambre fluidique de réception du liquide à caractériser, une source de lumière propre à éclairer la chambre suivant une direction longitudinale, un photodétecteur matriciel d'acquisition d'images du rayonnement transmis par la chambre éclairée et une unité de traitement d'informations,
- la figure 2 est une représentation très schématique du système de caractérisation. suivant un autre agencement de la source de lumière par rapport au photodétecteur matriciel,
- la figure 3 est une vue schématique de la chambre fluidique selon la direction longitudinale, ainsi que de l'agencement du photodétecteur matriciel de la figure 1 par rapport à la chambre, selon une première variante,
- la figure 4 est une vue analogue à celle de la figure 3 selon une deuxième variante,
- la figure 5 est un organigramme d'un procédé de caractérisation.
- la figure 6 est une image d'un canal vide de la chambre de la figure 1, acquise par le photodétecteur matriciel,
- les figures 7 et 8 sont des images de la chambre contenant le liquide, acquises par le photodétecteur à différents instants temporels, ces figures ne correspondant pas à l'objet des revendications annexées.
- les figures 9 et 10 sont des images de corrélation, calculées par l'unité de traitement de la figure 1, à partir des images acquises à différents instants temporels, ces figures ne correspondant pas à l'objet des revendications annexées.

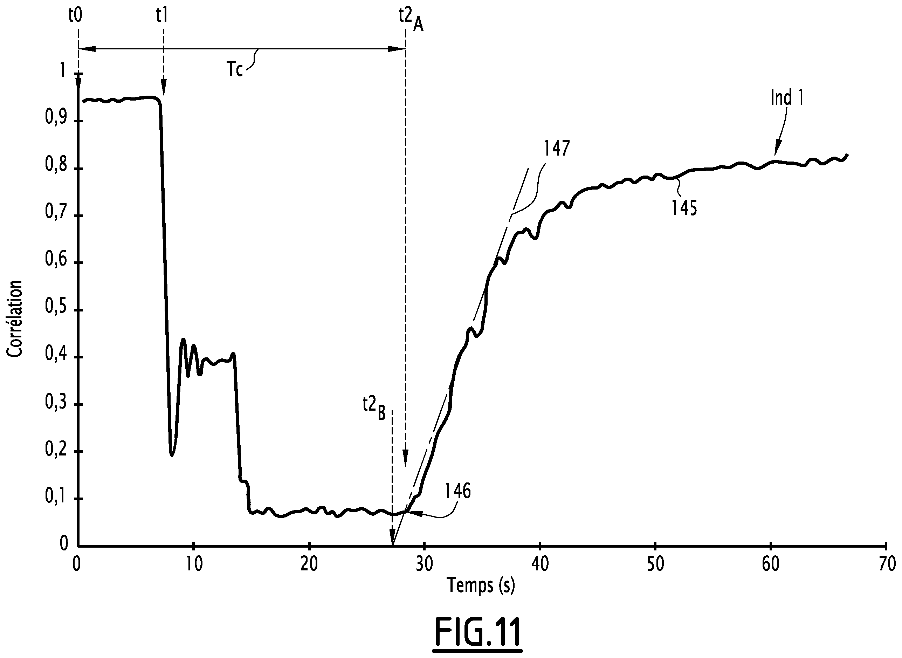

- la figure 11 est une représentation de l'évolution temporelle d'un indicateur caractérisant une variation de la vitesse des particules contenues dans le liquide, telle qu'un ralentissement des particules, cette figure ne correspondant pas à l'objet des revendications annexées.
- la figure 12 est un tableau illustrant les cas d'agrégations cellulaires en fonction du groupe sanguin et de l'anticorps déposés,
- la figure 13 est une vue analogue celle des figures 3 et 4 selon un deuxième mode de réalisation, la chambre fluidique comportant deux canaux,
- les figures 14 et 15 sont des images respectives des premier et deuxième canaux de la chambre de la figure 13, le deuxième canal présentant une agrégation cellulaire, les images étant acquises par le photodétecteur de la figure 1,
- les figures 16 et 17 sont des histogrammes du niveau de gris des images acquises des figures 14 et 15,
- les figures 18 à 21 sont des images du liquide à caractériser, acquises par le photodétecteur selon un deuxième exemple du deuxième mode de réalisation, le liquide à caractériser contenant du sang, auquel une quantité variable d'anticorps est ajoutée, ces images étant acquises pour des quantités croissantes d'anticorps,
- les figures 22 à 25 sont des histogrammes du niveau de gris des images acquises des figures 18 à 21 respectivement,
- les figures 26 à 29 sont des images du liquide caractérisé sur les figures 18 à 21 respectivement, obtenues, après dilution, à l'aide d'un microscope et formant des images de référence,
- les figures 30 à 34 sont des images du liquide à caractériser, acquises par le photodétecteur selon un troisième exemple du deuxième mode de réalisation, le liquide à caractériser contenant du sang, une quantité variable de protéine A, et une même quantité d'anticorps ajoutée,
- les figures 35 à 39 sont des histogrammes du niveau de gris des images acquises des figures 30 à 34 respectivement,
- les figures 40 à 44 sont des images du liquide caractérisé sur les figures 30 à 34 respectivement, obtenues après dilution à l'aide d'un microscope et formant des images de référence, et
- la figure 45 est une représentation très schématique de l'agglutination des globules rouges et des protéines A à l'aide des anticorps.

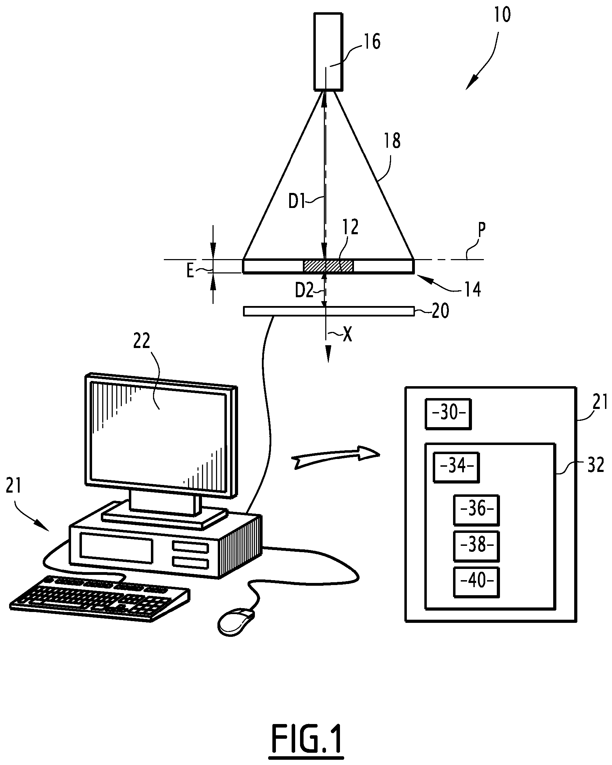

Sur la figure 1, un système de caractérisation 10 est destiné à caractériser une agglomération de particules, les particules, telles que des particules sanguines, étant contenues dans un liquide 12, via l'acquisition d'images formées par un rayonnement transmis par le liquide 12 éclairé, puis le traitement de ces images.

Ainsi, de façon générale, le système de caractérisation 10 est destiné à caractériser un paramètre d'un liquide comprenant des particules, ce liquide étant notamment du sang. Ce paramètre est, par exemple, une agglomération de particules constituant le liquide.

Par particule, on entend, par exemple, une particule biologique, c'est-à-dire une cellule (par exemple un globule rouge, un globule blanc, ou une plaquette), une bactérie, un virus ou encore toute autre molécule (par exemple une protéine).

Par agglutination (ou agglomération), on entend la formation d'un édifice tridimensionnel de particules reliées entre elles, sous l'effet d'un réactif introduit.

Par état d'agglutination (ou d'agglomération), on entend une estimation, relative ou absolue, de la taille des agglutinats ou relative à la quantité de particules présentes dans les agglutinats.

Le système de caractérisation 10 comprend une chambre fluidique 14 destinée à recevoir le liquide 12, une source de lumière 16 propre à émettre un faisceau lumineux d'excitation 18 pour éclairer la chambre fluidique 14, le faisceau lumineux 18 dirigé selon une direction longitudinale X à travers la chambre fluidique 14, et un photodétecteur matriciel 20 propre à acquérir des images du rayonnement transmis par la chambre fluidique 14 éclairée par le faisceau lumineux 18. Par rayonnement transmis, on entend le rayonnement traversant la chambre fluidique, de telle sorte que le photodétecteur matriciel 20 et la source de lumière 16 sont situés de part et d'autre de la chambre fluidique 14.

Le système de caractérisation 10 comprend une unité de traitement d'informations 21 et un écran 22 d'affichage d'une image de la chambre 14.

Dans le mode de réalisation décrit, le système de caractérisation 10 est propre à caractériser l'agglutination de particules sanguines, l'agglutination de particules sanguines permettant de déterminer le groupe sanguin associé. Le liquide 12 contient alors du sang. Le liquide 12 est, par exemple, du sang entier, une fraction du sang, ou encore un plasma sanguin. En variante, le liquide 12 est un autre liquide corporel, tel que l'urine, la sueur...

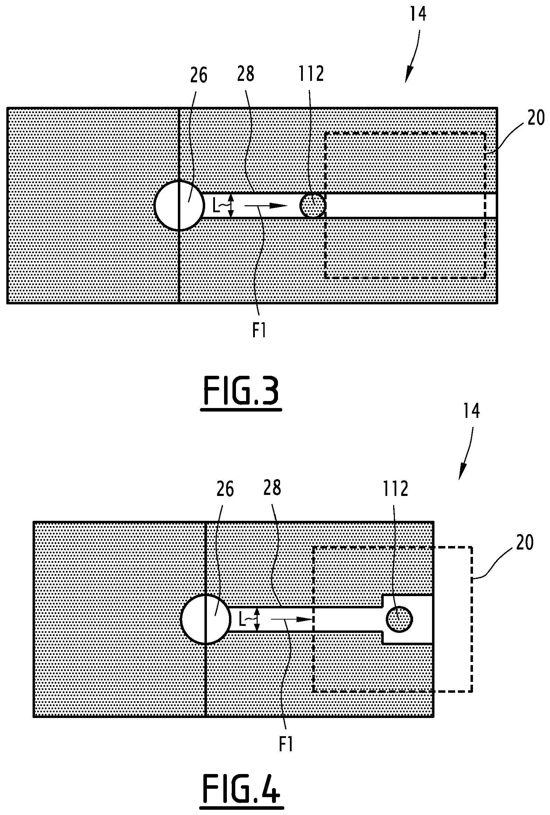

La chambre fluidique 14 est disposée entre la source de lumière 16 et le photodétecteur matriciel 20 selon la direction longitudinale X. La chambre fluidique 14 comprend une zone 26 de dépôt du liquide et un ou plusieurs canaux 28 de circulation du liquide 12, comme représenté sur la figure 3.

La chambre fluidique 14 comporte au moins un canal fluidique, délimité, selon la direction X, par une plaque supérieure et une plaque inférieure, non représentées. Ces plaques sont au moins partiellement translucides afin de permettre l'éclairement du liquide 12 par la source de lumière 16, ainsi que la détection du rayonnement transmis par le détecteur matriciel 20.

Les plaques inférieure et supérieure sont, par exemple, deux lames de verre, non représentées et séparées par des espaceurs non représentés, de sorte que les lames de verre sont espacées d'environ 160 $\mu$m selon la direction longitudinale X.

La chambre fluidique 14 présente une épaisseur E selon la direction longitudinale X. L'épaisseur E est, par exemple, de valeur comprise entre 20 $\mu$m et 1000 $\mu$m, de préférence comprise entre 30 $\mu$m et 300 $\mu$m.

La source de lumière 16 est propre à émettre le faisceau lumineux 18 selon la direction longitudinale X.

La source de lumière 16 est disposée à une première distance D1 de la chambre fluidique 14 selon la direction longitudinale X. La première distance D1 présente, de préférence, une valeur comprise entre 1 cm et 30 cm, par exemple égale à 20 cm.

Dans le mode de réalisation décrit, la source de lumière 16 est une source spatialement et temporellement cohérente. La source de lumière 16 est, par exemple, un laser. En variante, la source de lumière 16 est une diode Laser (DL) ou encore une diode Laser de type VCSEL (de l'anglais *Vertical Cavity Surface Emitting Laser*).

En variante encore, la source de lumière 16 est une diode électroluminescente, également appelée LED (de l'anglais *Light-Emitting Diode*), monochromatique et présentant des dimensions suffisamment réduites pour être considérée comme spatialement cohérente, le diamètre de la LED étant inférieur au dixième de la première distance D1 séparant cette LED de la chambre.

Le faisceau lumineux 18, orienté selon la direction longitudinale X, présente au niveau de la chambre fluidique, une surface comprise entre 5 mm$^2$ et 200 mm$^2$, de préférence égale à 25 mm$^2$, suivant un plan P perpendiculaire à la direction longitudinale X, comme représenté sur la figure 1. Le plan P est agencé au contact de la chambre fluidique 14. Ainsi, la surface de fluide éclairée est plus importante que dans l'état de la technique. Cela permet de s'affranchir de fluctuations locales du paramètre que l'on souhaite déterminer.

Le faisceau lumineux 18 est propre à éclairer directement la chambre fluidique 14, de préférence en l'absence d'une optique de grossissement disposée entre la source de lumière 16 et la chambre fluidique 14.

Le photodétecteur matriciel 20 est un capteur d'images pixélisé, comportant une pluralité de pixels, non représentés. Chaque pixel du photodétecteur 20 présente des dimensions inférieures ou égales à 10 $\mu$m, voire 4 $\mu$m. Chaque pixel est, par exemple, en forme d'un carré dont le côté est de valeur inférieure ou égale à 10 $\mu$m, voire 4 $\mu$m. Dans le mode de réalisation décrit, chaque pixel est en forme d'un carré de 4 $\mu$m de côté. En variante, chaque pixel est en forme d'un carré de 2,2 μm de côté.

Le photodétecteur matriciel 20 est disposé à une deuxième distance D2 de la chambre fluidique 14 selon la direction longitudinale X. La deuxième distance D2 présente une valeur inférieure à 1 cm, et de préférence comprise entre 100 μm et 2 mm. Le fait de privilégier une distance courte entre le détecteur et la chambre permet de limiter les phénomènes d'interférence entre les différentes figures de diffraction. En effet, lorsque cette distance augmente, ces interférences sont susceptibles de rendre l'image inexploitable, notamment lorsque le nombre de particules diffractantes augmente. Cela est dû au fait que, le volume de fluide éclairé est plus important que dans le dispositif décrit dans la demande EP 2 233 923 A1 de l'état de la technique. En plaçant le détecteur à une distance supérieure à 1cm, l'image obtenue sur le détecteur serait difficilement exploitable

Les images acquises par le photodétecteur matriciel 20 sont formées par le rayonnement transmis directement par la chambre fluidique 14 éclairée, en l'absence d'une optique de grossissement disposée entre la chambre fluidique 14 et le photodétecteur matriciel 20. Le photodétecteur matriciel 20 est également appelé dispositif d'imagerie sans lentille, et est apte à former une image de la chambre fluidique 14, tout en étant placé à une faible distance de cette dernière. Par faible distance, on entend une distance inférieure à 1 cm.

Le photodétecteur matriciel 20 est propre à générer au moins une image toutes les 5 secondes, et la cadence d'acquisition est donc supérieure à 0,2 Hz. Le photodétecteur matriciel 20 est un capteur d'images en deux dimensions, à savoir dans un plan perpendiculaire à l'axe longitudinal X. La fréquence d'acquisition des images est de préférence comprise entre 1 Hz et 20 Hz.

Le photodétecteur matriciel 20 est, par exemple, un capteur CCD. En variante, le photodétecteur 20 est un capteur CMOS.

Le photodétecteur matriciel 20 est, par exemple, sensiblement aligné avec la chambre fluidique 14 selon la direction longitudinale X, comme illustré sur la figure 3 où le photodétecteur 20 est représenté en traits pointillés.

En variante, le photodétecteur matriciel 20 est légèrement décalé par rapport à la chambre 14 suivant l'axe longitudinal X, comme illustré sur la figure 4 où le photodétecteur 20 est également représenté en traits pointillés.

L'unité de traitement d'informations 21, visible sur la figure 1, comporte un processeur de données 30 et une mémoire 32 associée au processeur.

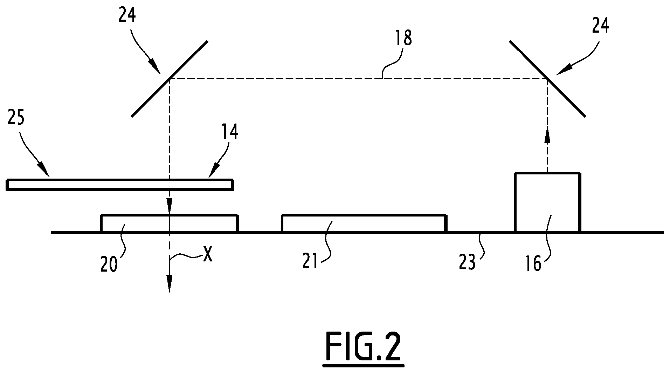

Dans l'exemple de réalisation de la figure 2, le photodétecteur matriciel 20, la source de lumière 16 et éventuellement tout ou partie de l'unité de traitement d'informations 21 sont solidaires d'un même substrat 23. Le système de caractérisation 10 comprend un système optique 24, par exemple un miroir, permettant de renvoyer le faisceau lumineux 18 de la source de lumière 16 vers le photodétecteur 20. Cela permet de disposer d'un système compact. La chambre fluidique 14 est, par exemple, ménagée dans un support amovible 25. Le support amovible 25 est, par exemple, jetable et est destiné à être inséré à l'aplomb du photodétecteur 20, à faible distance de ce dernier, de telle sorte que la chambre fluidique est apte à être éclairée par le faisceau lumineux 18. Selon cet exemple de réalisation, le support 25 est destiné à recevoir le fluide à analyser 12, puis à être inséré à proximité du photodétecteur 20 pour que l'analyse puisse être réalisée. Il comprend par exemple un conduit, dans lequel le fluide 12 circule jusqu'au canal 28 de la chambre fluidique, la chambre fluidique 14 étant raccordée à ce conduit. Lorsque l'analyse est terminée, le support 25 est retiré pour être, notamment, jeté. Le système de caractérisation 10 est alors disponible pour réaliser une autre mesure avec un autre support.

L'homme du métier comprendra que, dans l'exemple de réalisation de la figure 2, la direction longitudinale X correspond à la dernière portion du faisceau lumineux 18 entre le miroir correspondant du système optique 24 et le photodétecteur 20, traversant la chambre fluidique 14.

Le ou chaque canal de circulation 28 présente une largeur L, visible sur les figures 3 et 4. La largeur L est, par exemple, de valeur comprise entre 50 μm et 5 mm, de préférence égale à 1,5 mm.

La mémoire 32 est apte à stocker un logiciel 34 de réception des images acquises par le photodétecteur matriciel 20, un deuxième logiciel 38 de calcul d'un deuxième indicateur Ind2 apte à caractériser un autre paramètre recherché, en l'occurrence l'agglomération des particules. La mémoire 32 est également apte à stocker un logiciel 40 de caractérisation l'agglomération des particules.

En variante, les moyens de réception 34, les premiers moyens de calcul 36, les deuxièmes moyens de calcul 38 et les moyens de caractérisation 40 sont réalisés sous forme de composants logiques programmables ou encore sous forme de circuits intégrés dédiés.

Le logiciel de réception 34 est propre à recevoir régulièrement de la part du photodétecteur 20 les images acquises séquentiellement, à différents instants. Le logiciel de réception 34 est propre à recevoir au moins une image par seconde, et la cadence de réception des images est supérieure à 0,2 Hz, typiquement dans la plage de 1 Hz à 20 Hz.

Le premier logiciel de calcul 36, qui ne fait pas partie de l'invention, est propre à calculer une image $A_n$, représentant l'image de transmission $I_n(x,y)$, à laquelle est retranchée une moyenne locale. Cette dernière est obtenue en convoluant l'image $I_n(x,y)$ avec un noyau k1. Ce noyau k1 est une matrice de dimensions faibles par rapport à $I_n$. Par exemple, les dimensions du noyau k1 sont de 10 pixels par 10 pixels, et les dimensions de $I_n$ sont au moins deux fois supérieures à celles du noyau k1, voire 10 fois supérieures. Le noyau k1, comportant P lignes et Q colonnes, est, par exemple, homogène, toutes ses valeurs étant identiques. D'après ce qui précède, P et Q sont des nombres entiers, par exemple égaux à 10. Ainsi, on établit deux images $A_n$ et $A_{n+m}$, correspondant respectivement aux instants n et n+m, m étant un entier. En général, m est égal à 1, les images de transmission $I_n$ et $I_{n+1}$ étant deux images de transmission successives.

$$A_n(x, y) = I_n(x, y) - \left(I_n \otimes k1\right)(x, y) \tag{1}$$

$$A_{n+m}(x, y) = I_{n+m}(x, y) - \left(I_{n+m} \otimes k1\right)(x, y) \tag{2}$$

où $I_n(x, y)$, $I_{n+m}(x, y)$ représentent deux images de transmission successives aux instants n et n+m, x et y représentant les coordonnées d'un point de l'image respective, $I_n(x,y)$, $I_{n+m}(x,y)$ étant des matrices ayant X lignes et Y colonnes, le symbole $\otimes$ représentant le produit de convolution défini par l'équation suivante :

$$\left(F \otimes k1\right)(x, y) = \sum_{p=0}^{P} \sum_{q=0}^{Q} F(x - p, y - q) k1(p, q) \tag{3}$$

F étant une matrice à X lignes et Y colonnes,

k1 représentant un noyau pour la corrélation des images acquises, k1 étant une matrice à P lignes et Q colonnes,

X, Y, P et Q étant des nombres entiers vérifiant $X \geq P \geq 1$ et $Y \geq Q \geq 1$.

Les images sont, par exemple, acquises toutes les secondes par le photodétecteur matriciel 20, et les deux images de transmission $I_n(x,y)$, $I_{n+1}(x,y)$ sont alors des images acquises à une seconde d'intervalle.

Le logiciel de caractérisation 40 est propre à caractériser l'agglomération de particules contenues dans le liquide 12.

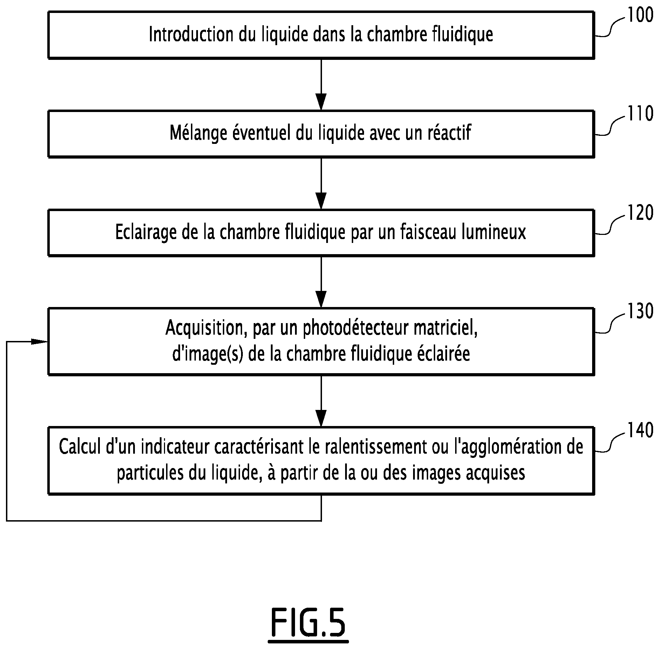

Le fonctionnement du système de caractérisation 10 selon l'invention va être à présent décrit à l'aide la figure 5 représentant un organigramme du procédé de caractérisation

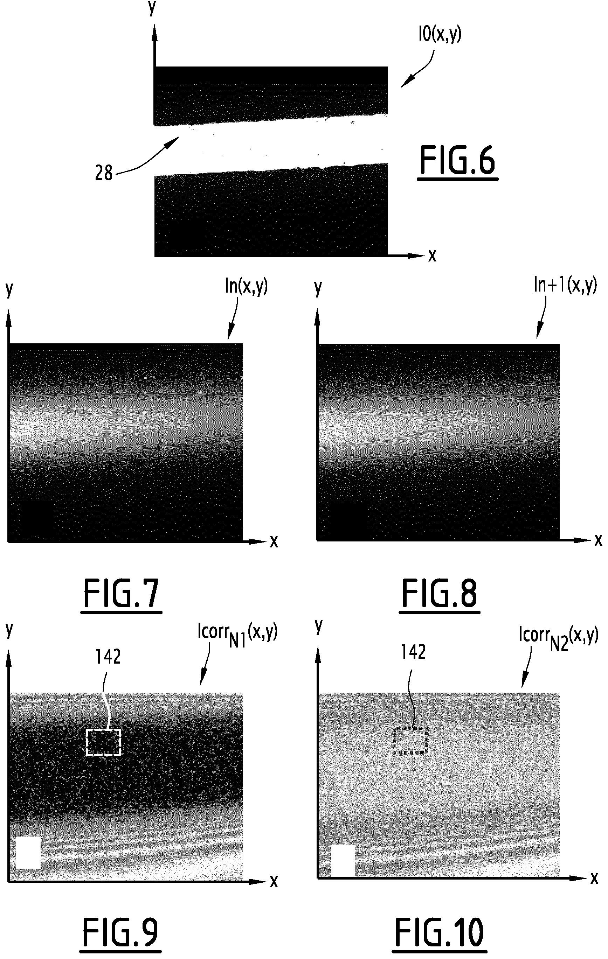

Préalablement à son utilisation, le ou les canaux de circulation 28 de la chambre fluidique sont vides, et une image initiale $I_0$ de la chambre 14 fait alors apparaître une zone blanche correspondant au canal de circulation 28 et des zones délimitant le canal, apparaissant dans cet exemple sous la forme de zones sombres correspondant au reste de la chambre fluidique 14, comme représenté sur la figure 6.

Lors de l'étape initiale 100, le liquide 12 est introduit dans la zone de dépôt 26 de la chambre fluidique. Le liquide 12 s'écoule par capillarité de la zone de dépôt 26 en direction du ou des canaux de circulation 28.

Le liquide 12 est ensuite éventuellement, lors de l'étape 110, mélangé avec un réactif 112, visible sur les figures 3 et 4, et apte à déclencher ou favoriser le phénomène de ralentissement des particules. Le réactif 112 est, par exemple, un réactif lyophilisé apte à favoriser le ralentissement des particules sanguines via une coagulation du sang.

Le réactif 112 est, par exemple, déposé en amont de la zone de détection optique correspondant à la zone à l'intérieur des pointillés sur la figure 3 pour laquelle une image est acquise par le photodétecteur 20. En variante, le réactif 112 est disposé à l'intérieur de la zone de détection optique, comme représenté sur la figure 4. Le mélange entre le liquide 12 et le réactif 112 s'effectue lorsque le liquide 12 s'écoule au contact du réactif 112 à l'intérieur du canal de circulation 28 (flèche F1).

Le liquide 12 est éclairé par le faisceau lumineux 18 lors de l'étape 120. La source de lumière 16 émet en effet le faisceau lumineux 18 en direction de la chambre fluidique 14 dans laquelle se trouve le liquide 12 selon la direction longitudinale X.

Lors de l'étape 130, le photodétecteur matriciel 20 effectue alors l'acquisition séquentielle de plusieurs images de transmission $I_n(x,y)$, $I_{n+m}(x,y)$ à des instants différents n et n+m. Chaque image de transmission $I_n(x,y)$, $I_{n+m}(x,y)$ est formée par le rayonnement transmis, à l'instant d'acquisition correspondant, par la chambre fluidique 14 éclairée.

Les images $I_n(x,y)$, $I_{n+m}(x,y)$ sont, par exemple, des images immédiatement successives, m étant alors égal à 1, de préférence acquises toutes les secondes, comme représentées sur les figures 7 et 8, où l'écart temporel entre les deux images $I_n(x,y)$, $I_{n+1}(x,y)$ acquises successivement est égal à une seconde.

Les images acquises $I_n(x,y)$, $I_{n+1}(x,y)$ correspondent aux interférences de figures de diffraction engendrées par des particules en suspension dans le liquide 12. L'éclairement des particules par le faisceau 18 spatialement et temporellement cohérent, tel qu'un faisceau laser, engendre une figure de diffraction, qui varie dans le temps à cause du mouvement des particules contenues dans le liquide 12.

L'observation d'une figure de diffraction exploitable, en plaçant le photodétecteur matriciel 20 à une distance aussi faible est notamment due à l'absence d'optique de grossissement entre la chambre fluidique 14 et le photodétecteur 20.

Lors de l'étape d'acquisition 130, le photodétecteur 20 est disposé à une faible distance de la chambre fluidique 14, la deuxième distance D2 entre la chambre fluidique 14 et le photodétecteur 20 selon la direction longitudinale X étant inférieure à 1cm.

L'étape 140 est décrite par la suite.

La deuxième distance D2 inférieure à 1cm entre la chambre fluidique 14 et le photodétecteur 20 permet également de limiter l'encombrement du système de caractérisation 10.

En outre, l'étendue importante du faisceau lumineux 18 suivant le plan P, c'est-à-dire supérieure à 5mm$^2$, et par exemple comprise entre 5mm$^2$ et 200mm$^2$, permet de limiter l'échauffement du liquide 12 contenu dans la chambre fluidique 14. En effet, la surface importante du faisceau lumineux 18 permet d'avoir une densité optique de puissance faible.

De plus, le fait d'utiliser un faisceau lumineux étendu et de former une image à faible distance de la chambre permet d'examiner un volume de fluide d'autant plus important On limite alors l'influence de phénomènes locaux, susceptibles de devenir prédominants lorsque le faisceau lumineux est plus fin, et le volume de fluide analysé est quasiment ponctuel. L'analyse de la corrélation entre deux images de transmissions $I_n$, $I_{n+m}$ permet de tenir compte de la structure spatiale de la coagulation, dans le plan du canal microfluidique 28. Autrement dit, on observe l'évolution de la coagulation du sang en deux dimensions.

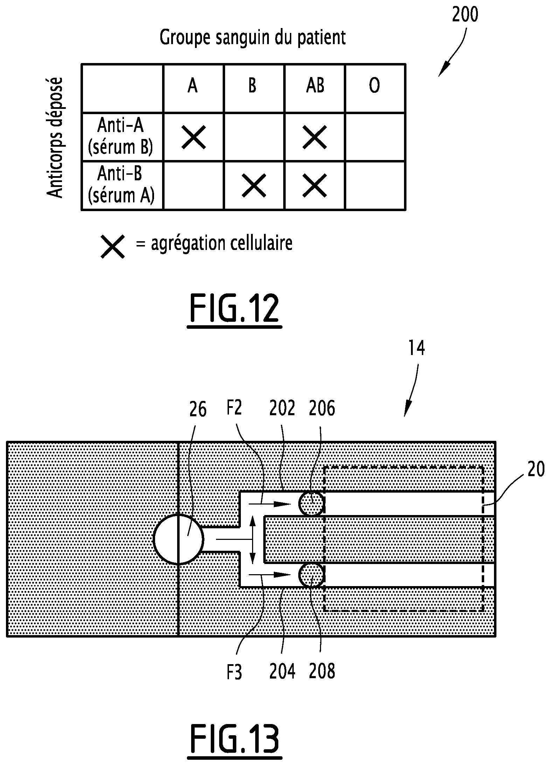

Les figures 12 à 17 illustrent un deuxième mode de réalisation, faisant l'objet de l'invention telle que revendiquée dans les revendications annexées, pour lequel les éléments analogues au premier mode de réalisation, décrit précédemment, sont repérés par des références identiques, et ne sont pas décrits à nouveau.

Selon le deuxième mode de réalisation, le système de caractérisation 10 est destiné à caractériser plus particulièrement l'agglomération de particules contenues dans un liquide 12. Le système de caractérisation 10 est, par exemple, apte à caractériser l'agglomération de particules sanguines, telles que des globules rouges, également appelée agglutination de particules sanguines.

Une information relative au groupe sanguin est alors en outre déterminée à partir de l'état d'agglomération, également appelé état d'agglutination.

Comme connu en soi, le groupe sanguin est déterminable selon le test de Beth-Vincent en détectant la présence d'antigènes A ou B impliquant l'absence d'anticorps anti-A ou anti-B. Dans le cas où les hématies du sang testé présentent un antigène A ou B, un complexe antigène-anticorps va se former et conduire à une agrégation cellulaire comme rappelé dans le tableau 200, visible sur la figure 12.

La chambre fluidique 14 comporte deux canaux de circulation 202, 204 distincts, à savoir un premier canal 202 et un deuxième canal 204, comme représenté sur la figure 13.

Selon le deuxième mode de réalisation, la source de lumière 16 est tout type de source de lumière. La source de lumière 16 n'est pas nécessairement spatialement et temporellement cohérente.

Selon le deuxième mode de réalisation, le deuxième logiciel de calcul 38 est propre à calculer le deuxième indicateur Ind2 apte à caractériser l'agglomération des particules, le deuxième indicateur Ind2 étant un indicateur d'intensité pour chaque image acquise $I_n(x,y)$. Le deuxième indicateur Ind2 est représentatif de l'histogramme de l'intensité de chaque pixel dans l'image $I_n$, ou dans une région d'intérêt de cette dernière. On le détermine, par exemple, en mesurant l'intensité totale de l'image $I_n$ ou dans une région d'intérêt prédéterminée de l'image $I_n$, éventuellement après seuillage.

Le logiciel de caractérisation 40 est propre à déterminer ensuite un état d'agglomération des particules du liquide 12 à partir du deuxième indicateur calculé Ind2. L'état d'agglomération est, par exemple, déterminé lorsque le deuxième indicateur Ind2 dépasse un seuil prédéterminé.

Dans le mode de réalisation décrit, où le liquide 12 contient du sang, les particules sont, par exemple, des globules rouges, et le logiciel de caractérisation est alors propre à déterminer une information relative au groupe sanguin à partir de l'état d'agglomération.

Le fonctionnement de ce deuxième mode de réalisation va être à présent décrit à l'aide des figures 13 à 17.

Lors de l'étape initiale 100, le liquide 12, par exemple l'échantillon sanguin d'un donneur dont on veut déterminer le groupe sanguin, est introduit dans la zone de dépôt 26 de la chambre fluidique. Le liquide 12 s'écoule alors de la zone de dépôt 26 en direction des canaux de circulation 202, 204, par exemple par capillarité.

Le liquide 12 est ensuite mélangé avec un premier 206 et un deuxième 208 réactifs distincts, lors de l'étape 110, comme représenté sur la figure 13.

Chaque réactif 206, 208 est, par exemple, déposé en amont de la zone de détection optique correspondant à la zone à l'intérieur des pointillés sur la figure 13 pour laquelle une image est acquise par le photodétecteur 20.

Le mélange entre le liquide 12 et les premier et deuxième réactifs 206, 208 s'effectue lorsque le liquide 12 s'écoule au contact du premier réactif 206 à l'intérieur du premier canal 202 (flèche F2), et respectivement du deuxième réactif 208 à l'intérieur du deuxième canal 204 (flèche F3).

Dans le mode de réalisation décrit, le premier réactif 206 est un sérum de donneur A, c'est-à-dire contenant des anticorps anti-B, et le deuxième réactif 208 est un sérum de donneur B, c'est-à-dire contenant des anticorps anti-A.

En fonction du groupe sanguin associé à l'échantillon sanguin 12, une agrégation cellulaire va alors se produire ou non dans chacun des canaux de circulation 202, 204.

Le liquide 12, tel que l'échantillon sanguin mélangé aux premier et deuxième réactifs 206, 208, est ensuite éclairé par le faisceau lumineux 18 lors de l'étape 120.

Lors de l'étape 130, le photodétecteur matriciel 20 effectue alors l'acquisition d'une image de transmission I(x,y) correspondant à une zone de détection optique englobant les deux canaux de circulation 202, 204.

L'homme du métier observera que, selon le deuxième mode de réalisation, l'acquisition d'une seule image de transmission I(x,y) permet de caractériser l'agglomération des particules contenues dans le liquide 12, en comparant cette image à une image de référence $I_{ref}(x,y)$, cette dernière étant par exemple une image réalisée sur une zone de référence, non représentée, dans laquelle le sang n'est pas mélangé à un réactif. Cette zone de référence est, par exemple, un troisième canal, de géométrie identique à celle du premier ou deuxième canal 202, 204, et ne comportant aucun réactif.

En variante, il s'agit d'une zone située sur le premier canal 202 ou sur le deuxième canal 204, en amont du réactif 206, 208.

En variante, l'image de référence $I_{ref}(x,y)$ est réalisée au même endroit que l'image de transmission, juste après le remplissage du canal par le liquide analysé, l'image de transmission I(x,y) étant réalisée, dans les mêmes conditions, après un certain temps, par exemple 1 minute, de telle sorte que l'effet éventuel du réactif sur le liquide analysé soit mesurable.

L'image acquise I(x,y) correspond de manière analogue à la diffraction et à la diffusion du faisceau lumineux 18 par les particules en suspension dans le liquide 12. De préférence, cette image est réalisée dans des conditions identiques pour les deux canaux, ainsi que pour la zone de référence. Par conditions identiques, on entend notamment les conditions d'éclairement, la distance source-détecteur, les caractéristiques du détecteur utilisé, le temps de pose, le champ observé, la taille de l'image.

L'éclairement des particules par le faisceau lumineux 18 engendre un motif de diffraction. Comme précédemment indiqué, l'absence de lentille de grossissement entre le fluide et le photodétecteur 20, couplée à la surface importante du faisceau incident, permet de former une image exploitable à faible distance, couvrant un champ important de fluide, tel qu'un champ présentant une aire de plusieurs mm$^2$.

Lors de l'étape d'acquisition 130, le photodétecteur 20 est disposé à proximité de la chambre fluidique 14, la deuxième distance D2 entre la chambre fluidique 14 et le photodétecteur 20 selon la direction longitudinale X étant inférieure à 1cm.

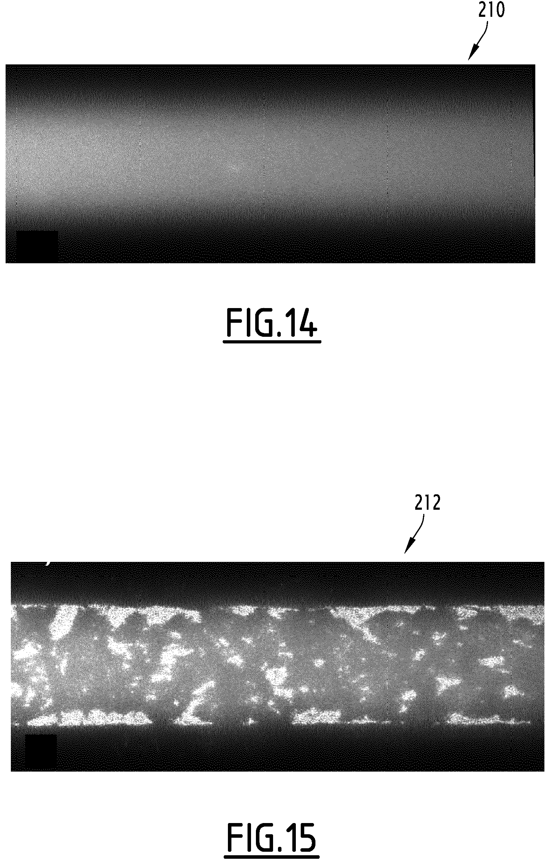

Dans l'exemple de réalisation des figures 14 et 15, représentant une image 210 acquise du premier canal 202, et respectivement une image 212 acquise du deuxième canal 204, une agrégation cellulaire est observée seulement dans le deuxième canal 204 de par la présence de tâches blanches dans l'image 212. Autrement dit, le groupe sanguin associé à l'échantillon sanguin testé est le groupe B d'après le tableau 200 de la figure 12.

A l'issue de l'étape d'acquisition 130, le deuxième logiciel de calcul 38 calcule, lors de l'étape 140, le deuxième indicateur Ind2 apte à caractériser l'agglomération des particules, le deuxième indicateur Ind2 étant un indicateur d'intensité pour chaque image acquise I(x,y). Le deuxième indicateur Ind2 est représentatif de l'intensité dans la région d'intérêt prédéterminée de l'image, en particulier de la distribution de l'intensité des pixels dans ladite région.

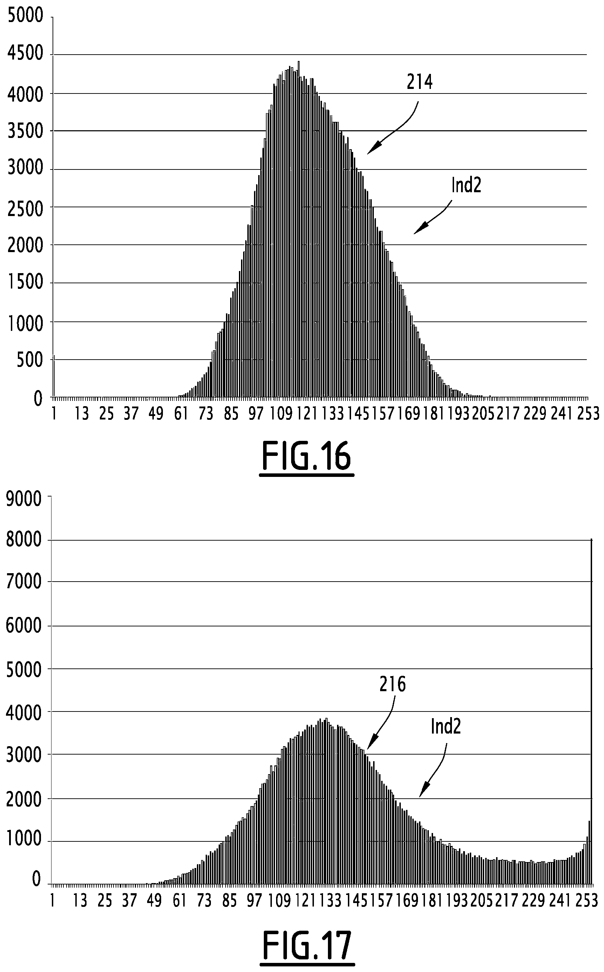

Le deuxième indicateur Ind2 est, par exemple, une caractéristique de l'image, et en particulier de l'histogramme du niveau de gris de l'image acquise pour chaque canal 202, 204, et le cas échéant, du canal de référence comme illustré sur les figures 16 et 17, représentant un histogramme 214 du niveau de gris de l'image acquise du premier canal 202, et respectivement un histogramme 216 du niveau de gris de l'image acquise du deuxième canal 204. Chaque histogramme de niveaux de gris 214, 216 présente en abscisse les valeurs de niveaux de gris et en ordonnée la population de pixels, c'est-à-dire le nombre de pixels, pour un niveau de gris donné en abscisse. Une caractéristique de l'histogramme est, par exemple, l'intensité moyenne des pixels, notée $I_{mean}$, après un éventuel seuillage de l'image, ce seuillage permettant de ne conserver que l'information des pixels dont l'intensité est supérieure à un certain seuil.

En se référant à l'exemple représenté sur les figures 16 et 17, après avoir effectué un seuillage à l'intensité correspondant à la valeur 120, on comprend que l'intensité moyenne de l'image correspondant à la figure 17, est supérieure à l'intensité moyenne de l'image correspondant à la figure 16. Cela vient du fait que l'histogramme de la figure 17, représentant l'observation d'une agglutination de particules, comprend davantage de pixels intenses (niveaux de gris supérieurs à 200) que l'histogramme de la figure 16, ce dernier représentant l'observation d'une non agglutination. Le deuxième indicateur Ind2 est alors, par exemple, établi selon l'intensité moyenne de l'image.

Selon une variante, on détermine, pour chaque image réalisée, l'intensité $I_{max}$, cette dernière correspondant, sur l'histogramme de l'image, à la valeur la plus élevée de l'intensité rassemblant un nombre prédéterminé de pixels, par exemple 500 pixels. On détermine ensuite l'écart entre $I_{max}$ et $I_{mean}$, par la soustraction $I_{max}-I_{mean}$, le deuxième indicateur Ind2 représentant alors cet écart. Sur l'histogramme de la figure 17, le deuxième indicateur Ind2 ainsi défini est plus élevé que sur l'histogramme de la figure 16. La valeur du deuxième indicateur Ind2 ainsi déterminée permet de conclure à la présence ou à l'absence d'un phénomène d'agglutination, via une comparaison, par exemple avec une valeur $Ind2_{ref}$ obtenue sur une zone de référence, ou encore avec une valeur prédéterminée, la prédétermination de cette valeur étant par exemple effectuée selon des essais expérimentaux.

Selon une variante, sur chaque image de transmission, on détermine l'intensité $I_{peak}$ correspondant à la valeur maximale de l'histogramme, c'est-à-dire la valeur d'intensité rassemblant le nombre le plus élevé de pixels. Sur les figures 16 et 17, cette valeur correspond au pic de chaque distribution, respectivement égales à 120 et 130. On détermine également la valeur maximum $I_{max}$ rassemblant un nombre de pixels de valeur supérieure à un seuil prédéterminé. En se référant aux figures 16 et 17, et en adoptant un seuil de 500, $I_{max}$ est respectivement égale à 181 et 256. Le deuxième indicateur Ind2 correspond à la distance entre $I_{max}$ et $I_{peak}$, respectivement 61 pour la figure 16 et 126 pour la figure 17. On conclut à une agglutination lorsque le deuxième indicateur Ind2 est supérieur, par exemple de 25%, à un certain seuil prédéterminé, ou lorsqu'il est supérieur à l'indicateur $Ind2_{ref}$ établi pour la zone de référence.

Selon une variante, le deuxième indicateur Ind2 est un indicateur de comparaison entre une région d'intérêt d'une image de transmission I et une image de référence $I_{ref}$ ne contenant pas de réactif (et donc dans lequel l'agglutination ne se produit pas) comme ci-dessous :

$$Ind2 \; = \frac{\sum_x \sum_y \left| I(x,y) - I_{ref}(x,y) \right|}{\sum_x \sum_y I(x,y) + I_{ref}(x,y)}$$

On compare le deuxième indicateur Ind2 avec un seuil prédéterminé, par exemple 0,25. Ainsi, si le deuxième indicateur Ind2 est supérieur à ce seuil; on conclut à une agglutination.

Le logiciel de caractérisation 40 est propre à déterminer ensuite un état d'agglomération des particules du liquide 12 à partir du deuxième indicateur calculé Ind2.

L'état d'agglomération est, par exemple, déterminé lorsque le deuxième indicateur Ind2 dépasse un seuil prédéterminé.

Si la comparaison est positive, c'est-à-dire si le niveau de gris obtenu est supérieur au seuil prédéterminé, alors le logiciel de caractérisation 40 en déduit la présence d'une agrégation cellulaire dans le canal 202, 204 correspondant.

Dans le deuxième mode de réalisation décrit, le logiciel de caractérisation 40 détermine enfin le groupe sanguin associé à l'échantillon sanguin 12 testé à partir du type des premier et deuxième réactifs 206, 208, ainsi que du tableau 200.

Les avantages de ce deuxième mode de réalisation sont identiques à ceux du premier mode de réalisation décrit précédemment.

En complément du premier mode de réalisation, la chambre fluidique 14 comporte une pluralité de canaux, par exemple les deux canaux 202, 204 visibles sur la figure 13, afin de caractériser la variation de la vitesse des particules du liquide 12 lorsque le liquide 12 est mélangé avec des réactifs différents, un réactif respectif étant alors disposé dans chaque canal 202, 204 de la chambre fluidique 14. Cela permet, avec un même dispositif, de déterminer différents paramètres d'analyse d'un même échantillon liquide, par exemple le temps de coagulation et le groupe sanguin.

Une telle chambre fluidique 14 est, par exemple, avantageuse pour caractériser la coagulation du liquide 12 contenant du sang, avec différents réactifs aptes à favoriser le ralentissement des particules sanguines via une coagulation du sang, tels que les différents réactifs 112 définis ci-dessus.

On conçoit ainsi que le système de caractérisation 10 selon l'invention permet d'observer une plus grande partie de la chambre fluidique 14, tout en ayant un encombrement limité.

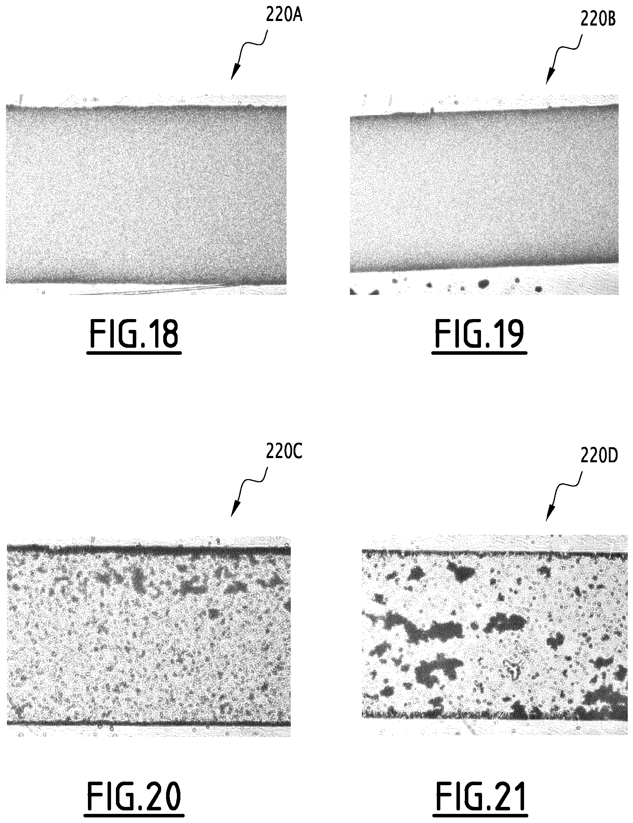

Les figures 18 à 29 illustrent un deuxième exemple du deuxième mode de réalisation, dans lequel le liquide à caractériser 12 est un liquide biologique, en particulier du sang ou du sang dilué, et le système de caractérisation 10 est apte à caractériser l'agglomération de particules, en l'occurrence des globules rouges dans le liquide biologique 12.

Le liquide à caractériser 12 comporte, dans cet exemple, du sang dilué au 1/20 dans un tampon PBS (de l'anglais *Phosphate Buffered Saline*), le tampon comportant 1% en volume de FBS (de l'anglais *Fœtal Bovine Serum*).

Le volume de sang dilué est de 40 $\mu$l, auquel on ajoute une quantité variable d'anticorps, tel qu'un anti-globule rouge appelé CD235A, commercialisé à titre d'exemple par la société Becton Dickinson sous la référence BD 555569. La quantité d'anticorps ajoutée varie de 0 à 1 $\mu$g d'anticorps par $\mu$l de sang non dilué, ce qui correspond à une concentration comprise entre 0 et 6.7 $\mu$M..

L'ajout de cet anticorps permet de masquer les antigènes de surface des globules rouges (en particulier la glycophorine A), ce qui entraîne leur agglutination.

L'objectif de ce deuxième exemple est de montrer qu'il est possible de caractériser un état d'agglutination de particules sanguines, par exemple de globules rouges, par imagerie sans lentille à l'aide du système de caractérisation 10.

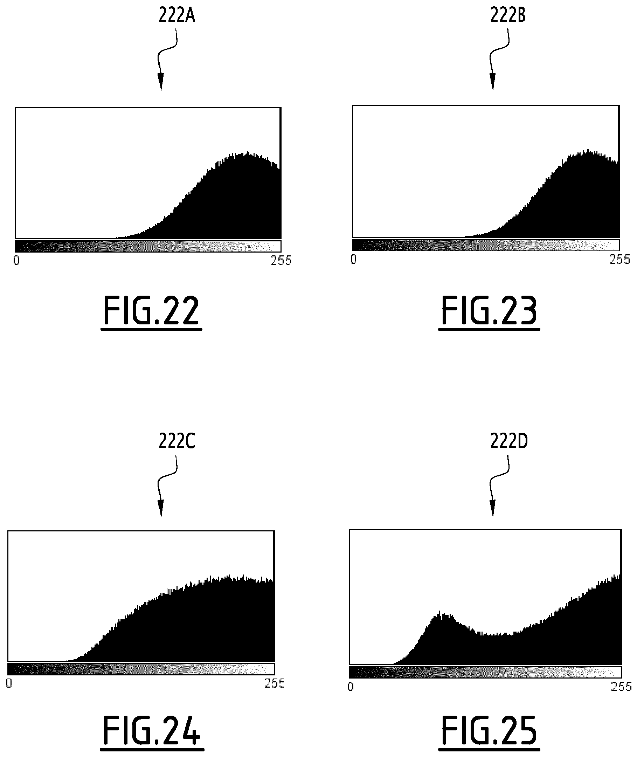

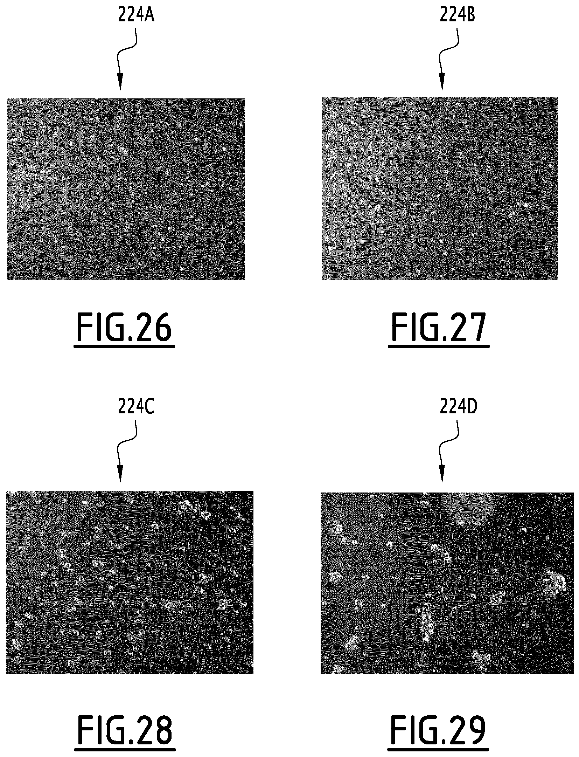

A chaque quantité d'anticorps ajoutée dans le liquide à caractériser 12, on effectue une acquisition de l'échantillon de liquide à caractériser 12 à l'aide du système de caractérisation 10, c'est-à-dire par imagerie sans lentille, les images 220A, 220B, 220C et 220D obtenues étant visibles sur les figures 18 à 21. Un histogramme en niveau de gris de l'intensité des pixels de chacune ces images 220A, 220B, 220C et 220D est ensuite calculé, les histogrammes calculés 222A, 222B, 222C et 222D étant visibles sur les figures 22 à 25. Des images de référence 224A, 224B, 224C et 224D de l'échantillon de liquide à caractériser 12 sont également obtenues avec un microscope, comme représenté sur les figures 26 à 29. Il est à noter que, pour l'observation en microscopie, l'échantillon sanguin est dilué avec un facteur de dilution d'un dixième.

Dans ce deuxième exemple, la source de lumière 16 est une diode Laser, présentant un spectre d'émission centré sur une longueur d'onde $\lambda$ par exemple égale à 670 nm, et la première distance D1 est sensiblement égale à 8 cm. L'échantillon est confiné dans la chambre fluidique 14 comportant un canal 28 d'épaisseur 150 $\mu$m ménagé entre deux parois transparentes d'épaisseur 200 $\mu$m. Ces parois sont réalisées en matériau plastique, par exemple en matériau COP (de l'anglais *Cyclo Olefin Polymer*).

La chambre fluidique 14 est directement posée sur le capot de verre du photodétecteur matriciel 20, tel qu'un capteur CMOS, comportant 1280 * 1024 pixels, chaque pixel étant de dimension 5 $\mu$m x 5 $\mu$m, de telle sorte que la chambre fluidique 14 est disposée entre le capteur CMOS et la source de lumière 16. La deuxième distance D2 est alors de préférence inférieure à 1 cm, par exemple égale à 550 $\mu$m.

Les acquisitions d'images sont par exemple réalisées avec un temps de pose 5 ms, avec une image par acquisition. Les images 220A, 220B, 220C et 220D correspondent respectivement à une quantité d'anticorps ajoutée croissante. Plus précisément, les images 220A, 220B, 220C et 220D correspondent respectivement à :

- une quantité d'anticorps sensiblement nulle,
- une quantité d'anticorps inférieure à une concentration seuil C,
- une quantité d'anticorps égale à la concentration seuil C, et
- une quantité d'anticorps égale à 2 fois la concentration seuil C.

Lorsque la quantité d'anticorps ajoutée dépasse la concentration seuil C, les globules rouges s'agglomèrent et les images obtenues par imagerie sans lentille à l'aide du système de caractérisation 10 reflètent la taille des agglutinats. La valeur de la concentration seuil C est, par exemple, égale à 250 ng d'anticorps pour 1 $\mu$l de sang non dilué, ce qui correspond à 1,7 $\mu$M.

On observe que l'agglomération de globules rouges entraîne l'apparition de zones étendues claires (fort niveau de gris) délimitées par des zones sombres (faible niveau de gris). Cet effet de segmentation d'image selon des zones comportant plusieurs dizaines, voire centaines de pixels, de niveaux de gris comparables, peut être observée en comparant les images 220A (figure 18) ou 220B (figure 19), sur lesquelles aucune agglutination n'est observée, aux images 220C (figure 20) et 220D (figure 21), sur lesquelles on observe une agglutination. La présence ou l'absence d'une agglutination de particules observable sur les images 220A, 220B, 220C et 220D est confirmée par les observations au microscope représentées sur les images 224A (figure 26), 224B (figure 27), 224C (figure 28) et 224D (figure 29) respectivement. Cela se traduit par une évolution de l'histogramme de chaque image, ce dernier tendant à s'étirer vers les faibles valeurs de niveau de gris au fur et à mesure que la quantité d'agglomérats augmente, comme cela est visible depuis l'histogramme 222A correspondant à l'image 220A en allant vers l'histogramme 222D correspondant à l'image 220D.

L'état d'agglutination de l'échantillon sanguin est alors quantifié par le calcul du deuxième indicateur Ind2 selon plusieurs variantes possibles :

- le deuxième indicateur Ind2 est, selon une première variante, égal à l'écart-type de la distribution en intensité des pixels de la zone d'intérêt examinée, et est alors noté $Ind2_A$,
- le deuxième indicateur Ind2 est, selon une deuxième variante, égal au nombre de pixels en deçà d'un certain seuil, ce seuil étant par exemple une fraction du niveau de gris maximal, divisé par le nombre total de pixels dans la zone d'intérêt examinée, et le deuxième indicateur Ind2 calculé selon cette deuxième variante est alors noté $Ind2_B$. Dans l'exemple des figures 22 à 25, la valeur du seuil est égale à 125.

Le tableau 1, ci-dessous, présente la valeur du deuxième indicateur Ind2 selon ces deux variantes et pour chacune des zones d'intérêt représentées sur les figures 18 à 21.

| Figures | 18 | 19 | 20 | 21 |
|---|---|---|---|---|
| $Ind2_A$ | 36 | 33 | 51 | 59 |
| $Ind2_B$ | $6.5 \ 10^{-3}$ | $6.5 \ 10^{-3}$ | $8.7 \ 10^{-2}$ | $1.4 \ 10^{-1}$ |

Lorsque le deuxième indicateur $Ind2_A$ selon la première variante est inférieur à une valeur seuil, comprise par exemple entre 40 et 45, il n'y a pas d'agglutination observable. Au-delà de cette valeur seuil, plus la valeur du deuxième indicateur $Ind2_A$ est élevée, plus la quantité de particules agglutinées est importante.

Le deuxième indicateur $Ind2_B$, calculé selon la deuxième variante, permet d'aboutir aux mêmes conclusions, en prenant une valeur seuil comprise entre $1\ 10^{-2}$ et $5\ 10^{-2}$.

Ainsi, on constate qu'il est possible d'observer, voire de quantifier, un état d'agglutination de particules dans le liquide biologique 12, à l'aide d'un indicateur calculé à partir d'une image obtenue par le système de caractérisation 10, c'est-à-dire par imagerie sans lentille, et notamment le deuxième indicateur $Ind2_A$, $Ind2_B$, selon les première et deuxième variantes de ce deuxième exemple, ce deuxième indicateur étant une fonction de la distribution de l'intensité des pixels des images 220A, 220B, 220C et 220D acquises par le système de caractérisation 10.

L'utilisation du système de caractérisation 10 est également envisageable dans un test diagnostic basé sur la détection d'agglutinats dans un fluide biologique.

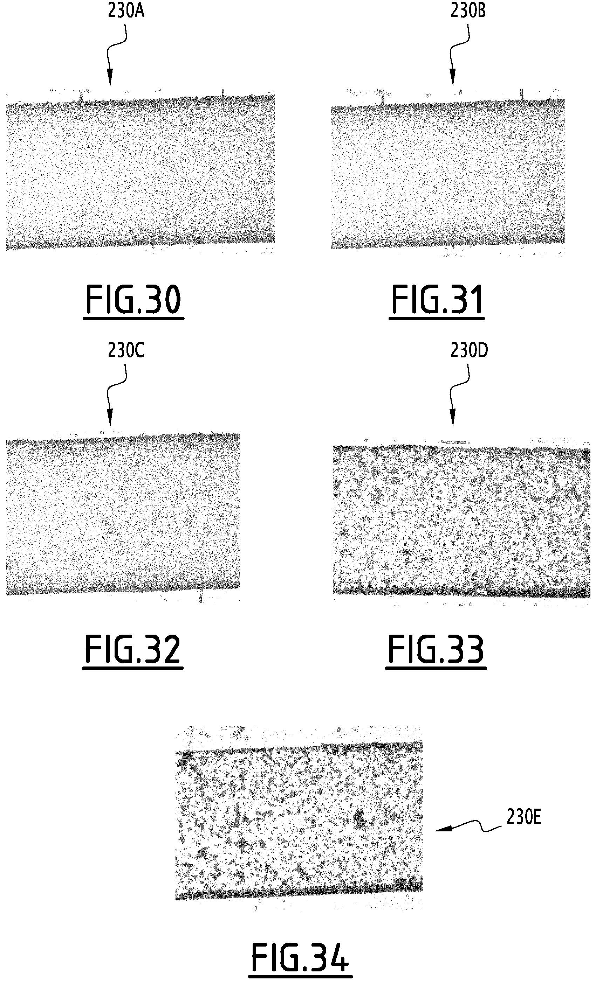

Les figures 30 à 45 illustrent un troisième exemple du deuxième mode de réalisation, dans lequel le liquide à caractériser 12 est un liquide biologique, en particulier du sang ou du sang dilué, et le système de caractérisation 10 est apte à caractériser l'agglomération, également appelé agglutination, de particules dans le liquide biologique 12.

Dans ce troisième exemple, on met en évidence la détection de l'agglutination de globules rouges dans un échantillon sanguin, comportant une quantité variable de protéine A, l'agglutination étant provoquée par l'ajout d'une quantité donnée d'un réactif (un anticorps).

Le liquide à caractériser 12 comporte, par exemple, du sang dilué au 1/20 dans un tampon PBS (de l'anglais *Phosphate Buffered Saline*), le tampon comportant 1% en volume de FBS (de l'anglais *Fœtal Bovine Serum*).

Le volume de sang dilué est de 40 µl, auquel on incube un anticorps, tel qu'un anti-globule rouge appelé CD235A, commercialisé à titre d'exemple par la société Becton Dickinson sous la référence BD 555569, avec une solution de protéine A en quantité variable. La durée d'incubation est de 1 heure.

Ainsi, on dispose de plusieurs solutions, dites anticorps - protéine A, dans lesquelles le ratio molaire anticorps - protéine A est variable. Ces solutions sont susceptibles d'entraîner l'agglutination de globules rouges, d'où la dénomination « solutions pro-agglutinantes ». Un volume d'1,2 µl de chacune de ces solutions est incubée avec 40 µl d'échantillon de sang dilué décrit ci-dessus, pendant 1,5 heures, chacun de ces mélanges formant un échantillon de liquide à caractériser 12.

Dans chacun des mélanges ainsi obtenu, la concentration molaire d'anticorps S est inférieure au seuil C déterminé dans le deuxième exemple précédent. Autrement dit, cette concentration d'anticorps ne permet pas l'agglutination spontanée de globules rouges. En l'occurrence, cette concentration S est de 100 ng d'anticorps par µl de sang non dilué, soit 0.7µM.

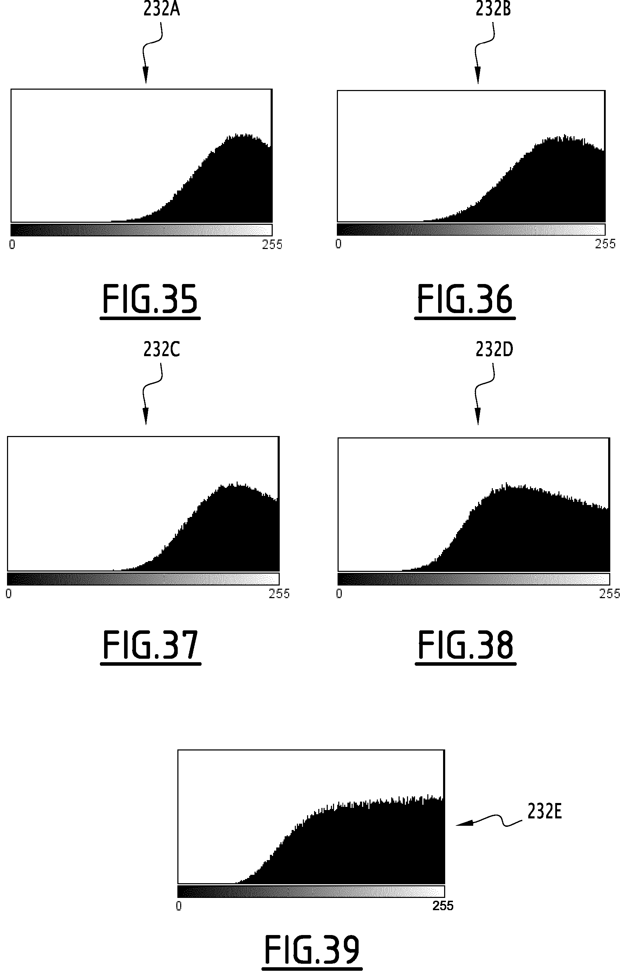

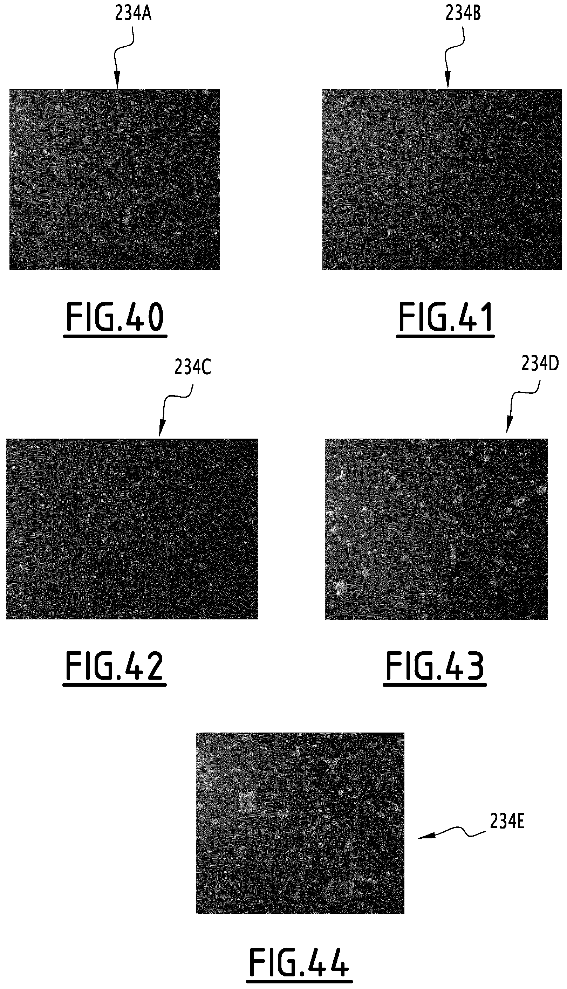

Pour chacun des échantillons de liquide à caractériser 12, on effectue une acquisition d'image à l'aide du système de caractérisation 10, c'est-à-dire par imagerie sans lentille, les images acquises 230A, 230B, 230C, 230D et 230E obtenues étant visibles sur les figures 30 à 34. Un histogramme en niveau de gris de l'intensité des pixels de chacune ces images 230A, 230B, 230C, 230D et 230E est ensuite calculé, les histogrammes calculés 232A, 232B, 232C, 232D et 232E étant visibles sur les figures 35 à 39. Des images de référence 234A, 234B, 234C, 234D et 234E de chacun des échantillons de liquide à caractériser 12 sont également obtenues avec un microscope, comme représenté sur les figures 40 à 44. Il est à noter que, pour l'observation en microscopie, l'échantillon sanguin est dilué avec un facteur de dilution d'un dixième.

Dans ce troisième exemple, la source de lumière 16 est une diode Laser, présentant un spectre d'émission centré sur une longueur d'onde λ égale à 670 nm, et la première distance D1 est sensiblement égale à 8 cm. L'échantillon est confiné dans la chambre fluidique 14 comportant un canal 28 d'épaisseur 150 µm ménagé entre deux parois transparentes d'épaisseur 200 µm. Ces parois sont réalisées en matériau plastique, par exemple en matériau COP (de l'anglais *Cyclo Olefin Polymer*).

La chambre fluidique 14 est directement posée sur le capot de verre du photodétecteur matriciel 20, tel qu'un capteur CMOS. Le capteur CMOS est par exemple une matrice 1280 par 1024 pixels, chaque pixel étant en forme d'un carré de 5 µm de côté ; de telle sorte que la chambre fluidique 14 est disposée entre le capteur CMOS et la source de lumière 16. La deuxième distance D2 est alors de préférence inférieure à 1 cm, par exemple égale à 550 µm.

Les acquisitions d'images sont par exemple réalisées avec un temps de pose 5 ms, avec une image par acquisition. Les images 230B, 230C, 230D et 230E correspondent respectivement à une quantité de protéine A ajoutée croissante. Plus précisément, les images 230A, 230B, 230C, 230D et 230E correspondent respectivement à :

- absence d'anticorps, soit un ratio molaire Anticorps : Protéine A = 0 : 40 (0 molécule d'anticorps pour 40 molécules de protéine A),
- absence de protéine A, soit un ratio molaire Anticorps - Protéine A = 1 : 0 (1 molécule d'anticorps pour 0 molécule de protéine A),
- ratio molaire Anticorps : Protéine A = 1 : 1 (1 molécule d'anticorps pour 1 molécule de protéine A),
- ratio molaire Anticorps : Protéine A = 1 : 5 (1 molécule d'anticorps pour 5 molécules de protéine A), et

- ratio molaire Anticorps : Protéine A = 1 : 40 (1 molécule d'anticorps pour 40 molécules de protéine A).

L'anticorps sert ici d'agent de couplage entre une molécule de protéine A et un globule rouge, comme cela sera détaillé ultérieurement.

En présence de protéine A et en l'absence d'anticorps, aucune agglutination de globules rouges n'est observée, comme représenté sur la figure 30. En présence d'anticorps et en l'absence de protéine A, aucune agglutination de globules rouges n'est observée, ceci étant visible sur la figure 31.

Lorsque le ratio anticorps : protéine A est égal à 1 : 1, aucune agglutination de globules rouges n'est également observée, comme représente sur la figure 32.

Lorsque le ratio anticorps : protéine A est égal à 1 : 5, on observe une agglutination de globules rouges, visible sur la figure 33. Lorsque le ratio anticorps : protéine A est égal à 1 : 40, on observe également une agglutination de globules rouges, visible sur la figure 34, la taille des agglutinats observés sur la figure 34 étant supérieure à celle des agglutinats observés sur la figure 33.

Les figures 35 à 39 représentent l'histogramme de l'intensité des pixels des figures 30 à 34 respectivement.

On observe que l'agglomération de globules rouges entraîne l'apparition de zones claires (fort niveau de gris) délimitées par une zone sombre (faible niveau de gris). Cet effet de segmentation d'image selon des zones comportant plusieurs dizaines, voire centaines de pixels, de niveaux de gris comparables, visible en comparant les images 230A (figure 30) ou 230B (figure 31) ou 230C (figure 32), sur lesquelles aucune agglutination n'est observée, aux images 230D (figure 33) et 230E (figure 34), sur lesquelles on observe une agglutination. La présence ou l'absence d'une agglutination de particules observable sur les images 230A, 230B, 230C, 230D et 230E est confirmée par les observations au microscope représentées sur les images 234A (figure 40), 234B (figure 41), 234C (figure 42), 234D (figure 43) et 234E (figure 44) respectivement. Cela se traduit par une évolution de l'histogramme de chaque image, ce dernier tendant à s'étirer vers les faibles valeurs de niveau de gris au fur et à mesure que la quantité d'agglomérats augmente, comme cela est visible depuis l'histogramme 232A correspondant à l'image 230A en allant vers l'histogramme 232E correspondant à l'image 230E. L'état d'agglutination de l'échantillon sanguin est alors quantifié par le calcul du deuxième indicateur Ind2 selon plusieurs variantes possibles :

- le deuxième indicateur Ind2 est, selon une première variante, égal à l'écart-type de la distribution en intensité des pixels de la zone d'intérêt examinée, et est alors noté $Ind2_A$,
- le deuxième indicateur Ind2 est, selon une deuxième variante, égal au nombre de pixels en deçà d'un certain seuil, ce seuil étant par exemple une fraction du niveau de gris maximal, divisé par le nombre total de pixels dans la zone d'intérêt examinée, et le deuxième indicateur Ind2 calculé selon cette deuxième variante est alors noté $Ind2_B$. Dans l'exemple des figures 22 à 25, la valeur du seuil est par exemple égale à 125.

Le tableau 2, ci-dessous, présente la valeur du deuxième indicateur Ind2 selon ces deux variantes et pour chacune des zones d'intérêt représentées sur les figures 30 à 34.

| Figures | 30 | 31 | 32 | 33 | 34 |
|---|---|---|---|---|---|
| $Ind2_A$ | 33 | 39 | 35 | 50 | 52 |
| $Ind2_B$ | $4.0\ 10^{-3}$ | $1.5\ 10^{-2}$ | $5.5\ 10^{-3}$ | $6.5\ 10^{-2}$ | $9.5\ 10^{-2}$ |

Lorsque le deuxième indicateur $Ind2_A$ selon la première variante est inférieur à une valeur seuil, comprise par exemple entre 40 et 45, il n'y a pas d'agglutination observable. Au-delà de cette valeur seuil, plus la valeur du deuxième indicateur $Ind2_A$ est élevée, plus la quantité de particules agglutinées est importante.

Le deuxième indicateur $Ind2_B$, calculé selon la deuxième variante, permet d'aboutir aux mêmes conclusions, en prenant une valeur seuil comprise entre $1\ 10^{-2}$ et $5\ 10^{-2}$.

Ainsi, on constate qu'il est possible d'observer, voire de quantifier, un état d'agglutination de particules dans le liquide biologique 12, à l'aide d'un indicateur calculé à partir d'une image obtenue par le système de caractérisation 10, c'est-à-dire par imagerie sans lentille, et notamment le deuxième indicateur $Ind2_A$, $Ind2_B$, selon les première et deuxième variantes de ce deuxième exemple, qui est fonction de la distribution de l'intensité des pixels des images 230A, 230B, 230C, 230D et 230E acquises par le système de caractérisation 10.

En outre, plus la quantité de protéine A est importante, plus la taille des agglutinats est importante, la quantité d'anticorps ajoutée étant constante. Ainsi, le deuxième indicateur $Ind2_A$, $Ind2_B$ quantifiant l'état d'agglutination permet également de quantifier une quantité de protéine dans l'échantillon sanguin.

En fonction du ratio molaire anticorps-protéine A, les globules rouges s'agglomèrent et les images 230A, 230B, 230C, 230D et 230E obtenues par imagerie sans lentille reflètent la taille des agglutinats, c'est-à-dire un degré d'agglutination. On comprend alors qu'en introduisant une quantité déterminée d'anticorps dans l'échantillon sanguin, il est possible d'estimer la quantité de protéine A présente dans cet échantillon en fonction de l'état d'agglutination, c'est-à-dire en fonction du deuxième indicateur $Ind2_A$, $Ind2_B$ précédemment décrit.

Autrement dit, la quantité de protéine A, au-delà de laquelle on observe une agglutination, constitue la limite de détection d'un dosage de cette protéine dans échantillon sanguin, en introduisant une quantité donnée d'anticorps dans l'échantillon de liquide à caractériser 12.

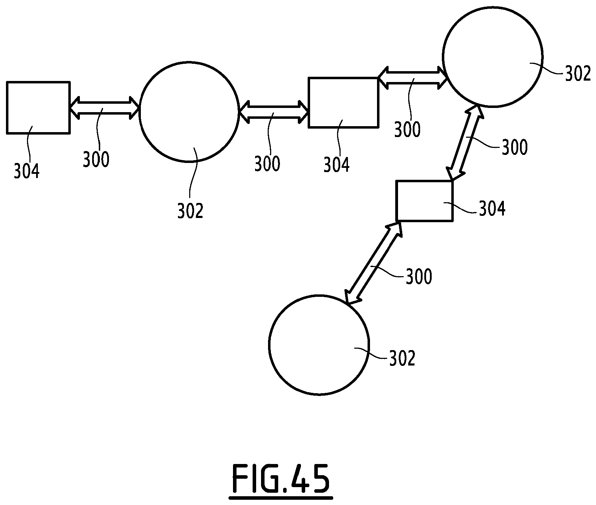

Ainsi, on comprend qu'il est possible d'observer, voire de quantifier, un état d'agglutination de particules dans un fluide biologique, par des indicateurs relatifs à l'image obtenue par imagerie sans lentille, et notamment le deuxième indicateur $Ind2_A$, $Ind2_B$, selon les première et deuxième variantes de ce deuxième exemple, qui est fonction de la distribution de l'intensité des pixels. Cet état d'agglutination dépend, par exemple, de la concentration d'un analyte dans le liquide biologique, la quantification de cet état d'agglutination permettant alors le dosage de cet analyte dans le liquide. L'exemple montre que ce dosage est réalisable en introduisant un réactif bi-fonctionnel, en l'occurrence l'anticorps 300, dans un échantillon sanguin, apte à se fixer à la fois sur une particule du fluide biologique, en l'occurrence les globules rouges 302, et sur l'analyte à doser, en l'occurrence la protéine A 304, formant ainsi un pontage entre un analyte 304 et les globules rouges 302, comme représenté sur la figure 45.

Le terme bi-fonctionnel désigne la faculté du réactif à se lier à la fois sur une particule et sur un analyte.

D'une façon générale, par analyte, on entend une espèce chimique ou biologique présente dans le liquide, telle qu'une molécule, une macromolécule (par exemple protéine ou acide nucléique), une cellule, une bactérie, un virus, ou encore un spore.

En outre, l'analyte 304 doit comporter au minimum 2 sites de liaison avec le réactif bi-fonctionnel, comme représenté sur la figure 45. Ainsi, chaque analyte 304 peut être lié, via le réactif bi-fonctionnel, à au moins deux particules. Cela entraîne l'agglutination des particules.

Autrement dit, l'état d'agglutination des particules dans le liquide 12 dépend de la quantité d'analyte présent dans le liquide 12, cette quantité étant dosable en ajoutant un réactif susceptible d'entraîner la formation d'agglutinats, le réactif 300 étant alors apte à se fixer entre une desdites particules 302 et un analyte 304 de façon à former un agglutinat.

En fonction de la quantité d'analyte 304 présent dans le liquide, on forme un agglutinat, composé de particules 302 et d'analytes 304. En déterminant l'état d'agglutination correspondant à une quantité donnée de réactif introduit, il est alors possible d'estimer la quantité d'analyte 304 présente dans le liquide 12.

Dans les deuxièmes et troisièmes exemples du deuxième mode de réalisation, décrits précédemment, la deuxième distance D2 est inférieure à 1 cm. Les inventeurs ont toutefois également observé que, dans le cas de la caractérisation de l'agglutination, des valeurs de la deuxième distance D2 supérieures à 1 cm, tels que des valeurs de quelques centimètres, voire quelques dizaines de centimètres, permettent d'obtenir des résultats exploitables, même si des valeurs de la deuxième distance D2 inférieures à 1 cm restent préférables.

D'une façon générale, ces deuxième et troisième exemples démontrent un autre aspect de l'invention. Selon cet autre aspect, l'invention concerne un procédé de caractérisation de l'agglutination de particules, telles des particules biologiques, dans un liquide, par exemple un liquide biologique, et en particulier un liquide corporel, le procédé de caractérisation comportant les étapes suivantes :

- l'introduction du liquide dans une chambre fluidique,
- l'éclairement de la chambre fluidique par un faisceau lumineux, le faisceau lumineux provenant en particulier d'une source de lumière, telle qu'une diode Laser ou une diode électroluminescente,
- l'acquisition d'une image, ou d'une pluralité d'images, de la chambre fluidique par un photodétecteur matriciel, le photodétecteur étant de préférence placé à une distance de la chambre fluidique inférieure à 1 cm, la chambre fluidique étant disposée entre la source de lumière et le photodétecteur matriciel,
- le traitement de l'image, ou de la pluralité d'images, pour déterminer un indicateur caractérisant l'agglutination de particules dans le liquide biologique, et
- la caractérisation de l'agglutination de particules dans le liquide, en fonction de la valeur de l'indicateur.

Il est à noter que l'image est acquise par le photodétecteur, de préférence sans optique de grossissement entre la chambre fluidique et le photodétecteur matriciel. Cependant, des microlentilles de focalisation peuvent être prévues au niveau de chaque pixel du détecteur, comme précédemment évoqué

En complément et de manière facultative, l'indicateur est un indicateur représentatif de la distribution de l'intensité des pixels dans une image, ou d'une façon plus générale, tout autre indicateur traduisant la segmentation de l'image selon différentes zones, chaque zone comportant plusieurs dizaines à centaines de pixels d'intensité comparable, c'est-à-dire dont l'intensité est répartie dans une plage de niveaux de gris de l'ordre de la moitié, voire du tiers, voire du quart, voire moins du quart de la dynamique de l'image.

En complément et de manière facultative, le procédé de caractérisation comporte l'ajout d'un réactif, susceptible d'entraîner l'agglutination de particules dans le liquide.

EP 2 821 786 B1

Comme cela est illustré dans le troisième exemple du deuxième mode de réalisation, l'agglutination des particules est, par exemple, fonction d'une quantité d'analyte présente dans le liquide.

Selon cette variante, l'invention concerne un procédé de détection de la quantité d'un analyte, dans un liquide, par exemple un liquide biologique, et en particulier un liquide corporel, le procédé de détection comportant les étapes suivantes :

- l'introduction du liquide dans une chambre fluidique,
- l'éclairement de la chambre fluidique par un faisceau lumineux, le faisceau lumineux provenant en particulier d'une source de lumière, telle qu'une diode Laser ou une diode électroluminescente,
- l'ajout d'un réactif, apte à entraîner la formation d'agglutinats de particules et d'analyte dans le liquide,
- l'acquisition d'une image, ou d'une pluralité d'images, de la chambre fluidique par un photodétecteur matriciel, le photodétecteur étant de préférence placé à une distance de la chambre fluidique inférieure à 1 cm, la chambre fluidique étant disposée entre la source de lumière et le photodétecteur matriciel,
- le traitement de l'image, ou de la pluralité d'images, pour déterminer un indicateur caractérisant l'agglutination de particules dans le liquide biologique, et
- l'estimation de la quantité d'analyte dans le liquide, en fonction de la valeur de l'indicateur.

Selon encore un autre aspect, l'invention concerne un procédé de détermination d'un paramètre du liquide 12, comportant du sang, le procédé comportant les étapes suivantes :

- l'introduction du liquide 12 dans la chambre fluidique 14,
- l'éclairage de la chambre fluidique 14 via le faisceau lumineux d'excitation 18 émis par la source de lumière 16, le faisceau lumineux 18 s'étendant à travers la chambre fluidique 14 selon la direction longitudinale X,
- l'acquisition d'au moins une image $I_n(x,y)$, $I_{n+m}(x,y)$, $I(x,y)$ par le photodétecteur matriciel 20, l'image $I_n(x,y)$, $I_{n+m}(x,y)$, $I(x,y)$ étant formée par un rayonnement transmis par la chambre fluidique 14 éclairée, et
- la détermination d'un indicateur $Ind1_{n,n+m}$, $Ind2$, à partir de ladite au moins une image $I_n(x,y)$, $I_{n+m}(x,y)$, $I(x,y)$.

Lors de l'étape d'acquisition, le photodétecteur 20 est disposé à la distance D2, inférieure à 1 cm, de la chambre fluidique 14 selon la direction longitudinale X.

En complément et de manière facultative, le procédé de détermination comprend une ou plusieurs des caractéristiques suivantes, prises isolément ou suivant toutes les combinaisons techniquement possibles :

- le faisceau lumineux 18 éclaire directement la chambre fluidique 14, et l'image $I_n(x,y)$, $I_{n+m}(x,y)$, $I(x,y)$ est formée directement par le rayonnement transmis par la chambre fluidique 14 éclairée, en l'absence d'une optique de grossissement disposée entre la chambre fluidique 14 et le photodétecteur 20 ;
- le paramètre est une coagulation, et le procédé comporte alors les étapes suivantes :

  + le mélange du liquide 12 avec un réactif pour favoriser la coagulation du sang,
  + l'acquisition d'une série d'image de transmission $I_n(x,y)$, $I_{n+m}(x,y)$ à des instants temporels n, n+m différents,
  + le calcul d'un indicateur $Ind1_{n,n+m}$ pour établir une corrélation entre deux zones des images de transmission $I_n(x,y)$, $I_{n+m}(x,y)$,

  la coagulation étant déterminée en fonction de la valeur dudit indicateur ;
- le paramètre est un temps de coagulation, et le procédé comporte alors les étapes suivantes :

  + le mélange du liquide 12 avec un réactif pour favoriser la coagulation du sang
  + l'acquisition d'une série d'image de transmission $I_n(x,y)$, $I_{n+m}(x,y)$ à des instants temporels n, n+m différents,
  + le calcul d'un indicateur $Ind1_{n,n+m}$ pour établir une corrélation entre deux images $I_n(x,y)$, $I_{n+m}(x,y)$, et
  + la détermination d'un intervalle temporel, dit temps de coagulation, entre un instant origine t0 et l'instant $t2_A$, $t2_B$ auquel l'indicateur $Ind1_{n,n+m}$ prend une valeur déterminée.

- le paramètre est une agglutination de particules sanguines, et le procédé comporte alors les étapes suivantes

  + le mélange du liquide 12 avec un réactif apte à engendrer une agglutination des particules sanguines,
  + l'acquisition d'une image de transmission $I(x,y)$,
  + le calcul d'un indicateur $Ind2$ en fonction de l'intensité dans une zone prédéterminée de l'image de transmission $I(x,y)$, et
  + la détermination d'un état d'agglutination lorsque cet indicateur $Ind2$ dépasse un seuil prédéterminé ;

- les particules sanguines sont des globules rouges, le réactif comportant un anticorps, l'état d'agglutination donnant alors une information relative au groupe sanguin.

Selon cet autre aspect indépendant, l'invention concerne également un système de détermination d'un paramètre du liquide 12, comportant du sang, le système de détermination comprenant :

- la chambre fluidique 14 destinée à recevoir le liquide 12;
- la source de lumière 16 propre à émettre le faisceau lumineux d'excitation 18 pour éclairer la chambre fluidique 14, le faisceau lumineux 18 s'étendant selon la direction longitudinale X;
- le photodétecteur matriciel 20 propre à acquérir au moins une image $I_n(x,y)$, $I_{n+1}(x,y)$, $I(x,y)$ d'un rayonnement transmis par la chambre fluidique 14 éclairée ; et
- l'unité de traitement d'informations 21 comportant des moyens de détermination d'un indicateur $Ind1_{n,n+m}$, $Ind2$, à partir de ladite au moins une image $I_n(x,y)$, $I_{n+m}(x,y)$, $I(x,y)$.

Le photodétecteur 20 est disposé à la distance D2, inférieure à 1 cm, de la chambre fluidique 14 selon la direction longitudinale X.

Le paramètre est une coagulation, un temps de coagulation, ou encore une agglutination de particules sanguines.

## Revendications

1.  Procédé de caractérisation de l'agglutination de particules, contenues dans un liquide (12), le procédé comportant les étapes suivantes :

    - l'introduction (100) du liquide (12) dans une chambre fluidique (14) ;
    - l'éclairement (120) de la chambre fluidique (14) via un faisceau lumineux (18) émis par une source de lumière (16), selon une direction longitudinale;
    - l'acquisition (130) d'une image $(I(x,y))$ ou d'une pluralité d'images $(I_n(x,y)$, $I_{n+m}(x,y))$ de la chambre fluidique (14) par un photodétecteur matriciel (20), la chambre fluidique (14) étant disposée entre la source de lumière (16) et le photodétecteur matriciel (20) ;
    - le traitement (140) de l'image $(I(x,y))$ ou de la pluralité d'images $((I_n(x,y)$, $I_{n+m}(x,y))$ pour déterminer un indicateur (Ind2) caractérisant l'agglutination de particules dans le liquide (12), et
    - la caractérisation de l'agglutination de particules dans le liquide (12) en fonction de la valeur de l'indicateur (Ind2) ;

    le procédé étant **caractérisé en ce que** lors de l'étape d'acquisition (130), le photodétecteur (20) est disposé à une distance (D2) inférieure à 1 cm de la chambre fluidique (14) selon la direction longitudinale (X).

2.  Procédé selon la revendication 1, dans lequel l'indicateur (Ind2) est représentatif de l'intensité des pixels de l'image $(I(x,y))$ dans une région prédéterminée de l'image $(I(x,y))$.

3.  Procédé selon la revendication 1 ou 2, dans lequel les particules sont des particules biologiques, telles que des particules sanguines.

4.  Procédé selon l'une quelconque des revendications précédentes, dans lequel la source de lumière (16) est une diode laser ou une diode électroluminescente.

5.  Procédé selon l'une quelconque des revendications précédentes, dans lequel le faisceau lumineux (18) éclaire directement la chambre fluidique (14), et l'image $(I_n(x,y)$, $I_{n+m}(x,y)$ ; $I(x,y))$ est formée directement par le rayonnement transmis par la chambre fluidique (14) éclairée, en l'absence d'une optique de grossissement disposée entre la chambre fluidique (14) et le photodétecteur (20).

6.  Procédé selon l'une quelconque des revendications précédentes, dans lequel l'agglutination est caractérisée lorsque l'indicateur (Ind2) dépasse un seuil prédéterminé.

7.  Procédé selon l'une quelconque des revendications 1 à 5, dans lequel l'état d'agglutination est déterminé lorsque l'indicateur (Ind2) dépasse un indicateur de référence $(Ind2_{ref})$, obtenu par une image réalisée dans une zone de référence.

8. Procédé selon l'une quelconque des revendications précédentes, dans lequel le procédé comporte en outre une étape (110) de mélange du liquide (12) avec un réactif (206, 208) apte à engendrer une agglutination des particules.

9. Procédé selon la revendication 8 et la revendication 3, dans lequel les particules sanguines sont des globules rouges, le réactif (206, 208) comporte un anticorps, et une information relative au groupe sanguin est en outre déterminée à partir de l'état d'agglutination.

10. Procédé selon l'une quelconque des revendications précédentes, dans lequel le liquide (12) comporte un analyte, le procédé comportant alors l'estimation de la quantité dudit analyte dans le liquide (12), en fonction de l'indicateur (Ind2).

11. Procédé selon l'une quelconque des revendications précédentes, dans lequel la chambre fluidique (14) comporte plusieurs canaux (202, 204) de circulation du fluide, et dans lequel, lors de l'étape de traitement (140), l'indicateur (Ind2) est calculé pour chacun des canaux (202, 204).

12. Système (10) de caractérisation de l'agglutination de particules contenues dans un liquide (12), le système (10) comprenant :

- une chambre fluidique (14) destinée à recevoir le liquide (12) ;
- une source de lumière (16) propre à émettre un faisceau lumineux d'excitation (18) pour éclairer la chambre fluidique (14), le faisceau lumineux (18) s'étendant à travers la chambre fluidique (14) selon une direction longitudinale (X) ;
- un photodétecteur matriciel (20) propre à acquérir une ou plusieurs images ($I_n(x,y)$, $I_{n+1}(x,y)$ ; $I(x,y)$) d'un rayonnement transmis par la chambre fluidique (14) éclairée ; et
- une unité de traitement d'informations (21) comportant des moyens de détermination (38), à partir de l'image ($I(x,y)$) ou de la pluralité d'images ($I_n(x,y)$, $I_{n+1}(x,y)$), d'un indicateur (Ind2) caractérisant l'agglutination de particules dans le liquide (12), et des moyens (40) de caractérisation de l'agglutination de particules dans le liquide (12) en fonction de la valeur de l'indicateur (Ind2), le système étant **caractérisé en ce que** le photodétecteur (20) est disposé à une distance (D2) inférieure à 1 cm de la chambre fluidique selon la direction longitudinale (X).

13. Système (10) selon la revendication 12, dans lequel le photodétecteur matriciel (20) comporte une pluralité de pixels, chaque pixel présentant des dimensions chacune inférieures ou égales à 4 $\mu$m.

**Patentansprüche**

1. Verfahren zur Charakterisierung der Agglutination von Partikeln, die in einer Flüssigkeit (12) enthalten sind, wobei das Verfahren die folgenden Schritte umfasst:

- Einleiten (100) der Flüssigkeit (12) in eine Fluidkammer (14);
- Beleuchten (120) der Fluidkammer (14) durch einen von einer Lichtquelle (16) ausgesendeten Lichtstrahl (18) in Längsrichtung;
- Erfassung (130) eines Bildes ($I(x,y)$) oder einer Vielzahl von Bildern ($I_n(x,y)$, $I_{n+m}(x,y)$) der Fluidkammer (14) durch einen Matrix-Fotodetektor (20), wobei die Fluidkammer (14) zwischen der Lichtquelle (16) und dem Matrix-Fotodetektor (20) angeordnet ist;
- die Verarbeitung (140) des Bildes ($I(x,y)$) oder der Vielzahl von Bildern (($I_n(x,y)$, $I_{n+m}(x,y)$) zur Bestimmung eines Indikators (Ind2), der die Agglutination von Partikeln in der Flüssigkeit (12) charakterisiert, und
- Charakterisierung der Agglutination von Partikeln in der Flüssigkeit (12) in Abhängigkeit des Wertes des Indikators (Ind2);

wobei das Verfahren **dadurch gekennzeichnet ist, dass** in der Erfassungsschritt (130) der Fotodetektor (20) in einem Abstand (D2) von weniger als 1 cm von der Fluidkammer (14) in Längsrichtung (X) angeordnet ist.

2. Verfahren nach Anspruch 1, wobei der Indikator (Ind2) die Intensität der Bildpunkte des Bildes ($I(x,y)$) in einem vorbestimmten Bereich des Bildes ($I(x,y)$) darstellt.

**3.** Verfahren nach Anspruch 1 oder 2, wobei die Partikel biologische Partikel, wie beispielsweise Blutpartikel, sind.

**4.** Verfahren nach einem der vorstehenden Ansprüche, wobei die Lichtquelle (16) eine Laserdiode oder eine Leuchtdiode ist.

**5.** Verfahren nach einem der vorstehenden Ansprüche, wobei der Lichtstrahl (18) die Fluidkammer (14) direkt beleuchtet und das Bild ($I_n$ (x,y), $I_{n+m}$ (x,y); I(x,y)) wird direkt durch die von der beleuchteten Fluidkammer (14) durchgelassene Strahlung gebildet, wobei keine Vergrößerungsoptik zwischen der Fluidkammer (14) und dem Fotodetektor (20) angeordnet ist.

**6.** Verfahren nach einem der vorstehenden Ansprüche, wobei der Agglutination **dadurch gekennzeichnet ist, dass** der Indikator (Ind2) einen vorbestimmten Schwellenwert überschreitet.

**7.** Verfahren nach einem der Ansprüche 1 bis 5, wobei der Zustand der Agglutination bestimmt wird, wenn der Indikator (Ind2) einen Referenzindikator ($Ind2_{ref}$) überschreitet, der durch ein in einem Referenzbereich erstelltes Bild erhalten wird.

**8.** Verfahren nach einem der vorstehenden Ansprüche, wobei das Verfahren zusätzlich einen Schritt (110) des Mischens der Flüssigkeit (12) mit einem Reagenz (206,208) umfasst, das eine Agglutination der Partikel bewirken kann.

**9.** Verfahren nach Anspruch 8 und Anspruch 3, wobei die Blutpartikel rote Blutkörperchen sind, das Reagens (206, 208) einen Antikörper enthält und eine Information bezüglich der Blutgruppe zusätzlich aus dem Agglutinationszustand bestimmt wird/

**10.** Verfahren nach einem der vorstehenden Ansprüche, wobei die Flüssigkeit (12) einen Analyten enthält, wobei das Verfahren dann die Abschätzung der Menge des Analyten in der Flüssigkeit (12) in Abhängigkeit vom Indikator (Ind2) umfasst.

**11.** Verfahren nach einem der vorstehenden Ansprüche, wobei die Fluidkammer (14) mehrere Kanäle (202, 204) für den Durchfluss des Fluids umfasst und wobei während des Verarbeitungsschritts (140) der Indikator (Ind2) für jeden der Kanäle (202, 204) berechnet wird.

**12.** System (10) zur Charakterisierung der Agglutination von in einer Flüssigkeit (12) enthaltenen Partikeln, wobei das System (10) umfasst:

- eine Fluidkammer (14) zur Aufnahme der Flüssigkeit (12);
- eine Lichtquelle (16), die einen Anregungslichtstrahl (18) aussenden kann, um die Fluidkammer (14) zu beleuchten, wobei sich der Lichtstrahl (18) in Längsrichtung (X) durch die Fluidkammer (14) erstreckt;
- einen Matrix-Fotodetektor (20), der ein Bild oder einer Vielzahl von Bildern ($I_n$ (x,y), $I_{n+1}$ (x,y); I(x,y)) einer von der beleuchteten Fluidkammer (14) durchgelassenen Strahlung erfassen kann; und
- eine Informationsverarbeitungseinheit (21) mit Mitteln zum Bestimmung (38) eines Indikators (Ind2), der die Agglutination von Partikeln in der Flüssigkeit (12) charakterisiert, aus dem Bild (I(x,y)) oder der Vielzahl von Bildern ($I_n$ (x,y), $I_{n+1}$ (x,y)), und mit Mitteln (40) zur Charakterisierung der Agglutination von Partikeln in der Flüssigkeit (12) in Abhängigkeit vom Wert des Indikators (Ind2) umfasst,

wobei das System **dadurch gekennzeichnet ist, dass** der Fotodetektor (20) in einem Abstand (D2) von weniger als 1 cm von der Fluidkammer in Längsrichtung (X) angeordnet ist.

**13.** System (10) gemäß Anspruch 12, wobei der Matrix-Fotodetektor (20) eine Vielzahl von Pixeln aufweist, wobei jedes Pixel Abmessungen von jeweils weniger als oder gleich 4 $\mu$m aufweist.

**Claims**

**1.** Method for characterising the agglutination of particles contained in a liquid (12), the method comprising the following steps:

- introducing (100) the liquid (12) into a fluid chamber (14);
- illuminating (120) the fluid chamber (14) via a light beam (18) emitted by a light source (16) in a longitudinal direction;
- acquiring (130) an image (I(x,y)) or a plurality of images ($I_n$(x,y), $I_{n+m}$(x,y)) of the fluid chamber (14) by a matrix photodetector (20), the fluid chamber (14) being arranged between the ight source (16) and the matrix photodetector (20);
- processing (140) the image (I(x,y)) or the plurality of images (($I_n$(x,y), $I_{n+m}$(x,y)) to determine an indicator (Ind2) characterising the agglutination of particles in the liquid (12), and
- characterising the agglutination of particles in the liquid (12) as a function of the value of the indicator (Ind2);

the method being **characterised in that** during the acquisition step (130), the photodetector (20) is positioned at a distance (D2) of less than 1 cm from the fluid chamber (14) in the longitudinal direction (X).

2. Method according to claim 1, wherein the indicator (Ind2) is representative of the intensity of the pixels of the image (I(x,y)) in a predetermined region of the image (I(x,y)).

3. Method according to claim 1 or 2, wherein the particles are biological particles, such as blood cells.

4. Method according to any of the preceding claims, wherein the light source (16) is a laser diode or a light-emitting diode.

5. Method according to any of the preceding claims, wherein the light beam (18) directly illuminates the fluid chamber (14), and the image ($I_n$(x,y), $I_{n+m}$(x,y); I(x,y)) is formed directly by the radiation transmitted through the illuminated fluid chamber (14), in the absence of a magnifying optical system arranged between the fluid chamber (14) and the photodetector (20).

6. Method according to any of the preceding claims, wherein agglutination is characterised when the indicator (Ind2) exceeds a predetermined threshold.

7. Method according to any of claims 1 to 5, wherein the state of agglutination is determined when the indicator (Ind2) exceeds a reference indicator ($Ind2_{ref}$), obtained from an image taken in a reference area.

8. Method according to any of the preceding claims, wherein the method further comprises a step (110) of mixing the liquid (12) with a reagent (206,208) capable of causing agglutination of the particles.

9. Method according to claim 8 and claim 3, wherein the blood particles are red blood cells, the reagent (206, 208) comprises an antibody, and information relating to the blood group is further determined from the agglutination state.

10. Method according to any of the preceding claims, wherein the liquid (12) comprises an analyte, the method then comprising estimating the amount of said analyte in the liquid (12) based on the indicator (Ind2).

11. Method according to any of the preceding claims, wherein the fluid chamber (14) comprises a plurality of fluid flow channels (202, 204), and wherein, during the processing step (140), the indicator (Ind2) is calculated for each of the channels (202, 204).

12. System (10) for characterising the agglutination of particles contained in a liquid (12), the system (10) comprising:

- a fluid chamber (14) for receiving the liquid (12);
- a light source (16) capable of emitting an excitation light beam (18) for illuminating the fluid chamber (14), the light beam (18) extending through the fluid chamber (14) in a longitudinal direction (X);
- a matrix photodetector (20) capable of acquiring one or more images ($I_n$(x,y), $I_{n+1}$(x,y); I(x,y)) of radiation transmitted through the illuminated fluidic chamber (14); and
- an information processing unit (21) comprising means (38) for determining, from the image (I(x,y)) or the plurality of images ($I_n$ (x,y), $I_{n+1}$ (x,y)), an indicator (Ind2) characterising the agglutination of particles in the liquid (12), and means (40) for characterising the agglutination of particles in the liquid (12) as a function of the value of the indicator (Ind2);
- the system being **characterised in that** the photodetector (20) is arranged at a distance (D2) of less than 1 cm from the fluidic chamber in the longitudinal direction (X).

13. System (10) according to claim 12, wherein the matrix photodetector (20) comprises a plurality of pixels, each pixel having dimensions each less than or equal to 4 $\mu$m.

## FIG.1

FIG.2

## FIG.3

## FIG.4

| | |
|---|---|
| Introduction du liquide dans la chambre fluidique | 100 |
| Mélange éventuel du liquide avec un réactif | 110 |
| Eclairage de la chambre fluidique par un faisceau lumineux | 120 |
| Acquisition, par un photodétecteur matriciel, d'image(s) de la chambre fluidique éclairée | 130 |
| Calcul d'un indicateur caractérisant le ralentissement ou l'agglomération de particules du liquide, à partir de la ou des images acquises | 140 |

## FIG.5

FIG.6

FIG.7

FIG.8

FIG.9

FIG.10

Corrélation

Ind 1

145

147

146

Temps (s)

**FIG.11**

t0  t1  t2$_A$  t2$_B$  Tc

Groupe sanguin du patient

| | A | B | AB | O |
|---|---|---|---|---|
| Anti-A (sérum B) | ✕ | | ✕ | |
| Anti-B (sérum A) | | ✕ | ✕ | |

200

Anticorps déposé

✕ = agrégation cellulaire

## FIG.12

14

26  F2  202  206

20

F3  204  208

## FIG.13

## FIG.14

## FIG.15

FIG.16

FIG.17

220A

## FIG.18

220B

## FIG.19

220C

## FIG.20

220D

## FIG.21

222A

FIG.22

222B

FIG.23

222C

FIG.24

222D

FIG.25

224A

FIG.26

224B

FIG.27

224C

FIG.28

224D

FIG.29

FIG.30

FIG.31

FIG.32

FIG.33

FIG.34

232A

FIG.35

232B

FIG.36

232C

FIG.37

232D

FIG.38

232E

FIG.39

234A

FIG.40

234B

FIG.41

234C

FIG.42

234D

FIG.43

234E

FIG.44

**FIG.45**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2233923 A1

**Littérature non-brevet citée dans la description**

- **PIEDERRIÈRE Y. et al.** *Evaluation of blood plasma coagulation dynamics by speckle analysis*
- **TRIPATHI M. et al.** *Assessing blood coagulation status with laser speckle rheology*
- **PIEDERRIÈRE Y. et al.** *Particle aggregation monitoring by speckle size measurement ; application to blood platelets aggregation*